(19) Europäisches Patentamt
European Patent Office
Office européen des brevets

(11) **EP 3 339 855 B1**

(12) **EUROPEAN PATENT SPECIFICATION**

(45) Date of publication and mention
of the grant of the patent:
**18.09.2019 Bulletin 2019/38**

(51) Int Cl.:
**G01N 33/00** *(2006.01)*     **G01N 1/22** *(2006.01)*

(21) Application number: **17196757.3**

(22) Date of filing: **17.10.2017**

(54) **SYSTEM AND METHOD FOR PREDICTING PRESENCE OF HAZARDOUS AIRBORNE MATERIALS IN A REGION TO BE PROTECTED**

SYSTEM UND VERFAHREN ZUR VORHERSAGE DES VORHANDENSEINS GEFÄHRLICHER STOFFE IN DER LUFT IN EINEM ZU SCHÜTZENDEN BEREICH

SYSTÈME ET PROCÉDÉ PERMETTANT DE PRÉDIRE LA PRÉSENCE DE MATIÈRES ATMOSPHÉRIQUES DANGEREUSES DANS UNE ZONE À PROTÉGER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.12.2016 IL 24978016**

(43) Date of publication of application:
**27.06.2018 Bulletin 2018/26**

(73) Proprietor: **Wolfson, Noah**
**6900209 Tel Aviv (IL)**

(72) Inventor: **Wolfson, Noah**
**6900209 Tel Aviv (IL)**

(74) Representative: **Evens, Paul Jonathan et al**
**Maguire Boss**
**24 East Street**
**St. Ives, Cambridgeshire PE27 5PD (GB)**

(56) References cited:
**US-A- 5 604 299     US-A1- 2008 195 329**
**US-A1- 2010 268 480**

• **John A Cooper ET AL: "Guide for Developing a Multi-Metals, Fence-Line Monitoring Plan for Fugitive Emissions Using X-Ray Based Monitors", , 3 December 2010 (2010-12-03), XP055471844, Retrieved from the Internet: URL:https://www3.epa.gov/ttnemc01/prelim/o tm31.pdf [retrieved on 2018-05-02]**

**Description**

REFERENCES TO RELATED APPLICATIONS

Reference is hereby made to Israel Patent No. 147725, entitled "Determining Location of Noxious Emissions," published July 24, 2007.

FIELD OF THE INVENTION

The present invention relates to dealing with prediction of the presence of hazardous airborne materials.

BACKGROUND OF THE INVENTION

A system and method of calculating and displaying plume arrival time of a Chemical, Biological or Radiological contaminant is described in U.S. Patent No. 9,110,748.

SUMMARY OF THE INVENTION

The present invention seeks to provide improved systems and methods for predicting presence of hazardous airborne materials in a region to be protected.

There is thus provided in accordance with a preferred embedment of the present invention a method for predicting presence of hazardous airborne materials in a region to be protected, the method including receiving an indication of an actual or possible hazardous airborne material emission at at least one emission location outside of the region to be protected and spaced therefrom by an intermediate region, ascertaining the at least one emission location, ascertaining a time of initial emission of the airborne material, ascertaining an emission rate of hazardous airborne material at the at least one emission location and based on measured wind pattern data taken at multiple mutually displaced wind measurement locations in the intermediate region, and at least the time of initial emission and the emission rate, calculating an expected concentration pattern over time of the hazardous airborne materials.

In accordance with a preferred embodiment of the present invention the receiving an indication includes measuring an actual concentration of the hazardous airborne material at a concentration measurement location displaced from the at least one emission location. Additionally or alternatively, the calculating an expected concentration pattern includes, based on measured wind pattern data taken at multiple mutually displaced wind measurement locations, calculating an axis of an emission plume of the hazardous airborne material from the each of the at least one emission location. Additionally, the calculating an expected concentration pattern further includes calculating at least one minimum distance of at least one concentration measurement location from the axis of the emission plume.

Preferably, the calculating an expected concentration pattern additionally includes generating iso-concentration lines.

In accordance with a preferred embodiment of the present invention the method also includes providing an alert to persons located or expected to be located within in a region to be protected and expected to encounter a concentration of the hazardous airborne material above a predetermined threshold.

In accordance with a preferred embodiment of the present invention the calculating an expected concentration pattern over time of the hazardous airborne materials includes calculating a gridded wind field based on current measured wind velocities and assuming the gridded wind field is persistent for an expected time of travel of the hazardous airborne materials to a protected populated area. Additionally or alternatively, the calculating an expected concentration pattern over time of the hazardous airborne materials includes measuring an actual concentration of the hazardous airborne material at at least one concentration measurement location displaced from the at least one emission location, calculating a back trajectory from each of the at least one concentration measurement location based on historic measured wind velocities, calculating a gridded wind field based on current measured wind velocities and assuming the gridded wind field is persistent for an expected time of travel of the hazardous airborne materials to a protected populated area.

Preferably, the ascertaining the at least one emission location includes measuring an actual concentration of the hazardous airborne material at at least one concentration measurement location displaced from the at least one emission location, calculating a back trajectory from each of the at least one concentration measurement location, calculating a minimum distance from each of the back trajectories to each of the at least one emission location and selecting an emission location corresponding to a lowest of the minimum distances. Additionally, the ascertaining a time of initial emission of the airborne material includes selecting a time along the back trajectory corresponding to the lowest of the minimum distances.

There is also provided in accordance with another preferred embodiment of the present invention a system for predicting presence of hazardous airborne materials in a region to be protected, the system including a communication module receiving an indication of an actual or possible hazardous airborne material emission at at least one emission location outside of the region to be protected and spaced therefrom by an intermediate region, an emission location ascertainer, ascertaining the at least one emission location, a time of initial emission ascertainer, ascertaining a time of initial emission of the airborne material, an emission rate ascertainer, ascertaining an emission rate of hazardous airborne material at the at least one emission location and an expected concentration pattern generator, operative, based on measured wind pattern data taken at multiple mutually displaced wind measurement locations in the intermediate region and at least the time of initial emission and the emission rate, to calculate an expected concentration pattern over time of the hazardous airborne materials.

In accordance with a preferred embodiment of the present invention the communication module is operative to receive an actual measured concentration of the hazardous airborne material at a concentration measurement location displaced from the at least one emission location.

Preferably, the expected concentration pattern generator is operative, based on measured wind pattern data taken at multiple mutually displaced wind measurement locations, to calculate an axis of an emission plume of the hazardous airborne material from the each of the at least one emission location. Additionally, the expected concentration pattern generator is operative to calculate at least one minimum distance of at least one concentration measurement location from the axis of the emission plume.

Preferably, the expected concentration pattern generator also includes an iso-concentration line generator.

In accordance with a preferred embodiment of the present invention the system for predicting presence of hazardous airborne materials also includes an alert generator operative to provide an alert to persons located or expected to be located within in a region to be protected and expected to encounter a concentration of the hazardous airborne material above a predetermined threshold.

There is also provided in accordance with another preferred embodiment of the present invention a method for indicating a source of a contaminant in a given region, the method including identifying at least one contaminant emission location outside of the given region and spaced therefrom by an intermediate region, detecting a concentration of the contaminant above a predetermined threshold in the region to be protected, based on measured wind pattern data taken at multiple mutually displaced wind measurement locations in the intermediate region, and at least the concentration, calculating a concentration pattern over time of the contaminant and indicating a source of the contaminant from one of the at least one contaminant emission location.

There is further provided in accordance with yet another preferred embodiment of the present invention a method for indicating a path of a contaminant from a contamination source to a given region, the method including identifying at least one contaminant emission location outside of the given region and spaced therefrom by an intermediate region, detecting a concentration of the contaminant above a predetermined threshold in the given region, based on measured wind pattern data taken at multiple mutually displaced wind measurement locations in the intermediate region, and at least the concentration, calculating a concentration pattern over time of the contaminant.

There is even further provided in accordance with still another preferred embodiment of the present invention a method for indicating a source of a hazardous airborne material in a region to be protected, the method including identifying at least one hazardous airborne material emission location outside of the region to be protected and spaced therefrom by an intermediate region, detecting a concentration of a hazardous airborne material above a predetermined threshold in the region to be protected, based on measured wind pattern data taken at multiple mutually displaced wind measurement locations in the intermediate region, and at least the concentration, calculating an expected concentration pattern over time of the hazardous airborne material and indicating a source of the hazardous airborne material from one of the at least one hazardous airborne material emission location.

There is still further provided in accordance with yet another preferred embodiment of the present invention a method for indicating a path of a hazardous airborne material from a contamination source to a region to be protected, the method including identifying at least one hazardous airborne material emission location outside of the region to be protected and spaced therefrom by an intermediate region, detecting a concentration of the hazardous airborne material above a predetermined threshold in the region to be protected, based on measured wind pattern data taken at multiple mutually displaced wind measurement locations in the intermediate region, and at least the concentration, calculating an expected concentration pattern over time of the hazardous airborne material.

BRIEF DESCRIPTION OF PREFERRED EMBODIMENTS

The present invention will be understood and appreciated more fully from the following detailed description, taken in conjunction with the drawings in which:

Fig. 1 is a simplified illustration of a system for prediction and alert of concentration pattern of hazardous airborne materials constructed and operative in accordance with a preferred embodiment of the present invention;
Fig. 2 is a simplified flowchart illustrating principal steps in a methodology employing the system of Fig. 1;

Figs. 3A, 3B, 3C, 3D, 3E are simplified overlay illustrations showing progressive steps in the methodology of Fig. 2 employing the system of Fig. 1;

Fig. 4 is a simplified illustration of a system for prediction and alert of concentration pattern of hazardous airborne materials constructed and operative in accordance with another preferred embodiment of the present invention;

Figs. 5A and 5B, taken together, are a simplified flowchart illustrating principal steps in a methodology employing the system of Fig. 4;

Figs. 6A, 6B, 6C and 6D are simplified overlay illustrations showing progressive steps in the methodology of Figs. 5A and 5B employing the system of Fig. 4;

Fig. 7 is a simplified illustration of a system for prediction and alert of concentration pattern of hazardous airborne materials constructed and operative in accordance with another preferred embodiment of the present invention;

Figs. 8A and 8B, taken together, are a simplified flowchart illustrating principal steps in a methodology employing the system of Fig. 7; and

Figs. 9A, 9B, 9C and 9D are simplified overlay illustrations showing progressive steps in the methodology of Figs. 8A and 8B employing the system of Fig. 7.

DETAILED DESCRIPTION OF PREFERRED EMBODIMENTS

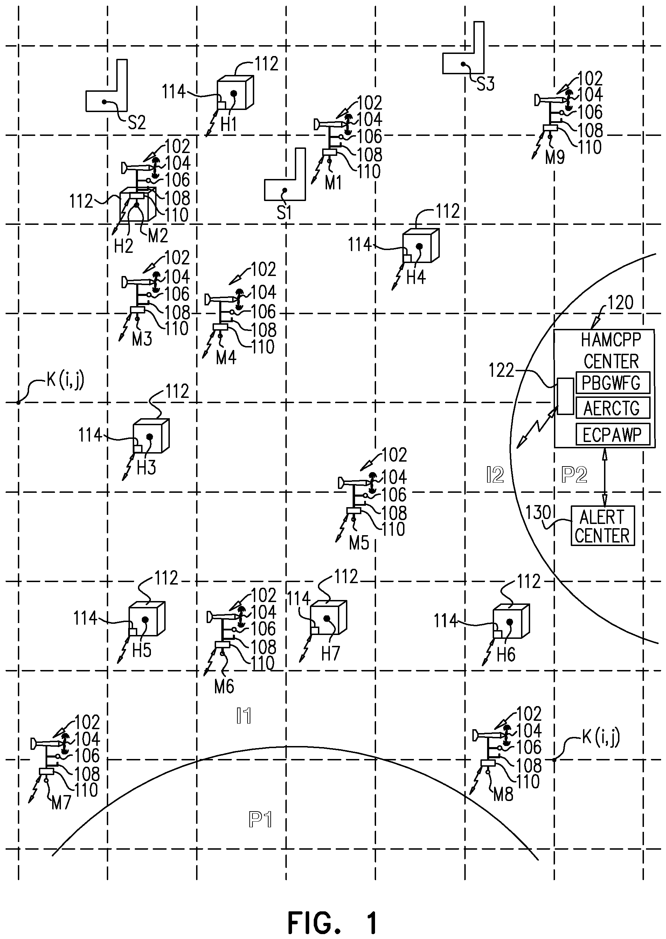

Reference is now made to Fig. 1, which is a simplified illustration of a system for prediction of the presence of hazardous airborne materials, constructed and operative in accordance with a preferred embodiment of the present invention.

As seen in Fig. 1, the system preferably operates within a geographical region to be monitored which includes multiple known potential sources of known hazardous airborne materials, for example, an ammonium plant, an oil refinery, a food additives plant, a pharmaceutical plant, a nuclear power plant, a compost plant and a sewage treatment plant, whose locations are known and are designated by respective designators S1, S2, S3 ... Sn, populated areas to be protected, here designated PI, P2 ... Pn, and intermediate regions, here designated I1, I2, ... In. It is appreciated that known source locations need not necessarily be fixed locations, for example, a known location of a derailed tanker. For the purposes of the description which follows, it is assumed that the locations S1, S2, S3 are outside of the populated areas to be protected, PI, P2, and separated therefrom by one or more intermediate regions I1, 12.

It is appreciated that not all populated areas are populated areas to be protected. Whether a given populated area is considered to be a populated area to be protected depends, inter alia, on the propinquity of a given source S to the populated area and on the wind conditions at the time of the emission.

The system of Fig. 1 preferably employs a plurality of meteorological stations 102 at known locations in one or more intermediate region, here designated by respective designators M1, M2, M3 ... Mn. Each of the meteorological stations 102 preferably includes a wind velocity and direction measurement device 104 and a pyranometer 106 and may also include temperature, relative humidity and air pressure measurement devices, collectively designated by reference numeral 108. Each of the meteorological stations 102 preferably includes at least one bidirectional communications module 110, which enables communication to and from the meteorological stations 102. Additional meteorological stations 102 may also be located at the source locations S and within the populated areas P to be protected. Alternatively, meteorological data from some of meteorological stations 102 may be obviated by using computer weather forecast models of sufficient resolution and reliability.

The system of Fig. 1 preferably also includes a plurality of geographically dispersed hazardous airborne material sensor assemblies 112, at locations in the intermediate regions, each designated as H1, H2, H3 ... Hn. Hazardous airborne material sensor assemblies 112 may all be identical or, alternatively, different sensors may be specific to different hazardous airborne materials. Hazardous airborne material sensor assemblies 112 preferably each include at least one bidirectional communications module 114, which enables communication of sensor output information from the sensor assembly 112 within the system and also preferably enables remote control of the hazardous airborne material sensor assembly 112.

Sensor assemblies 112 may be co-located with meteorological stations 102 or may be stand-alone. Where a sensor assembly 112 is co-located with a meteorological station 102, communications module 114 may be obviated and the sensor assembly 112 may communicate via communications module 110. Sensor assemblies 112 preferably measure and report the concentrations of hazardous airborne material every one second.

Sensor assemblies 112 each preferably include one or more of the following sensor modules:

Manning EC-F9-NH3, commercially available from Honeywell Analytics, Inc. of 405 Barclay Blvd., Lincolnshire, IL 60069, USA;

Portable Multi-Gas Detector PGM-5020, commercially available from Rae Systems Inc. of 3775 North First Street, San Jose, CA 95134 USA, detecting volatile organic compounds (VOC);

Gas Chromatography Volatil Organic Compounds (BTEX) Analyzer VOC72M; commercially available from Environnement S.A, 111 boulevard Robespierre, 78300 Poissy, France, measuring, inter alia, Benzene, Toluene, Ethylbenzene, m+p-Xylene, o-Xylene, 1-3 Butadiene;

G460 Multi-Gas Detector, commercially available from GfG Europe, of Unit 8, Griggs Business Centre, West Street, Coggeshall, Essex, UK CO6 INT, sensing LEL, $O_2$, H2S, CO, $NH_3$, $SO_2$, $CL_2$, $PH_3$, $NO_2$, HCN, ETO, $ClO_2$, HF, $O_3$, NO and H2, as well as an infrared (NDIR) sensor for the direct measurement of carbon dioxide and a photoionization detector (PID) for the measurement of volatile organic compounds (VOCs);

AQM 65 air monitoring station, commercially available from Aeroqual Limited, of 109 Valley Road, Mount Eden, Auckland, New Zealand, measuring common air pollutants including ozone ($O_3$), nitrogen dioxide ($NO_2$), nitrogen oxides ($NO_x$), carbon monoxide (CO), Sulphur dioxide ($SO_2$), volatile organic compounds (VOC), hydrogen sulfide ($H_2S$), non-methane hydrocarbons (NMHC), carbon dioxide (CO2), particulate matter (TSP, PM10, PM2.5, PM1);

Ultra RAE 3000 Model PGM-7360, commercially available from Rae Systems, Inc. of 3775 North First Street, San Jose, CA 95134 USA, measuring VOC, benzene and butadiene (Photoionization sensor);

TA-2100 Toluene Gas Detector, commercially available from Mil-Ram Technology, Inc, of 48009 Fremont Boulevard, Fremont, CA 94538, USA, including PID Photo-Ionization sensor technology;

APSA-H370 monitor, commercially available from Horiba, Ltd. of 2, Miyanohigashi, Kisshoin, Minami-Ku Kyoto 601-8510 Japan, measuring sulfur dioxide ($SO_2$) and hydrogen sulfide ($H_2S$) using the oxidation catalyst and ultraviolet fluorescent (UVF) method as its operating principle;

FM-801 monitor, commercially available from Gray Wolf Sensing Solutions, LLC of 6 Research Drive, Shelton, CT 06484 USA, measuring formaldehyde concentration (Photoelectric Absorptiometry);

TA-2100 Styrene Monomer Gas Detector, commercially available from Mil-Ram Technology, Inc, of 48009 Fremont Boulevard, Fremont, CA 94538 USA, utilizing PID Photo-Ionization;

Model 405 nm NO2/NO/NOx Monitor, commercially available from 2B Technologies, of 2100 Central Avenue, Suite 105, Boulder, Colorado 80301 USA, utilizing direct absorbance of $NO_2$ at 405 nm;

EDM 765, commercially available from Grimm Aerosol Technik GmbH & Co. KG, of Dorfstr. 9, 83404 Ainring, Germany, including dust and gas monitoring system measuring fine dust and gases, for instance CO, $NO_x$, $O_3$;

LasIR RP101 Gas Analyzer, commercially available from Unisearch Associates Inc, of 96 Bradwick Drive, Concord, L4K 1K8 Ontario, Canada; measuring HF, NH3, CO/$CO_2$, $CH_4$, $H_2S$, HCl and

A-915M Mercury Analyzer, commercially available from Ohio Lumex, of 30350 Bruce Industrial Parkway, Solon, OH 44139, USA, utilizing atomic absorption spectrometry at 254 nm with Zeeman correction for background absorption for interference-free measurement.

In accordance with a preferred embodiment of the present invention, the system includes an Hazardous Airborne Material Concentration Pattern Prediction (HAMCPP) center 120, which may be at any suitable location, fixed or mobile, and may be wholly or partially implemented on the cloud.

HAMCPP center 120 preferably includes a Persistence Based Gridded Wind Field Generator (PBGWFG) module, an Assumed Emission Rate of hazardous airborne material at at least one emission location at Current Time Generator (AERCTG) module and a particle arrival time (PAT) and Expected Concentration Pattern Ascertainer employing current measured wind data and Assumed Wind Persistence (ECPAWP) module.

HAMCPP center 120 also includes one or more communication modules 122 and preferably communicates with the meteorological stations 102 and with the sensor assemblies 112 using any suitable communication technology and preferably provides an Hazardous Airborne Material Concentration Pattern Prediction (HAMCPP) and particle arrival time (PAT) output. The HAMCPP center 120 may also employ additional wind inputs from available computerized weather forecasting models and may integrate such inputs in its mean calculations which are used for computing the HAMCPP output.

The multiplicity of meteorological stations 102 are preferably distributed over the geographical region to be monitored. Typically, between eight and sixteen meteorological stations 102 are employed to cover an area between 28 x 28 km and 60 x 60 km. The optimal density of the meteorological stations 102 depends on local topographical and climatic conditions. Each meteorological station 102 preferably measures and logs the wind velocity, preferably measuring every one second and preferably providing an average wind velocity (AWV) over a predetermined duration, typically ranging from one to ten minutes, and logging moving average values and standard deviations of wind velocity as well as solar radiation intensity. Alternatively, as described below, HAMCPP center 120 calculates the average wind velocity from the meteorological stations 102 inputs.

Preferably, data communication from the sensor assemblies 112 to the HAMCPP center 120 is either initiated by a command from the HAMCPP center 120 or carried out automatically at preset times established by the HAMCPP center 120. Such preset times preferably correspond to times immediately following the logging of average values of wind velocity and their standard deviations as well as the solar radiation intensity.

The HAMCPP center 120 typically receives inputs via one or more communication modules 122 from the meteorological stations 102, typically including a wind velocity and direction measurement from wind velocity and direction measurement device 104, and may also receive inputs from hazardous airborne material sensor assemblies 112, typically every one second. In one embodiment of the present invention, HAMCPP center 120 provides a moving average of the wind velocities, AWV, over a predetermined duration, which may range from 1 - 10 minutes, and preferably outputs the moving average of the wind velocities AWV every one second. The output of HAMCPP center 120 preferably indicates the predicted pattern of one or more given concentrations of airborne hazardous materials in the geographical region to be monitored and possibly therebeyond. A typical HAMCPP output is one or more iso-concentration contour lines, overlaid over a map and indicating in which populated areas within the geographical region to be monitored a dangerous concentration of an airborne hazardous material is currently present or is expected to be present.

Preferably, the HAMCPP center 120 communicates the iso-concentration contours overlaid over a map, via its communication modules 122, to one or more population alert centers 130, each of which is responsible for the safety of the inhabitants of a given area. Each alert center 130 may be operated automatically, semiautomatically or manually to generate an appropriate alert to the relevant population.

Examples of alerts may be sirens, radio, television, sms, email and social network notifications or combinations thereof.

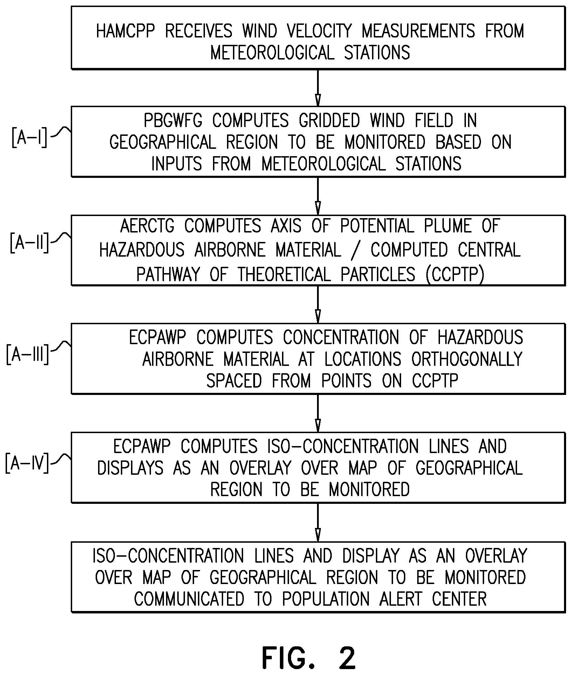

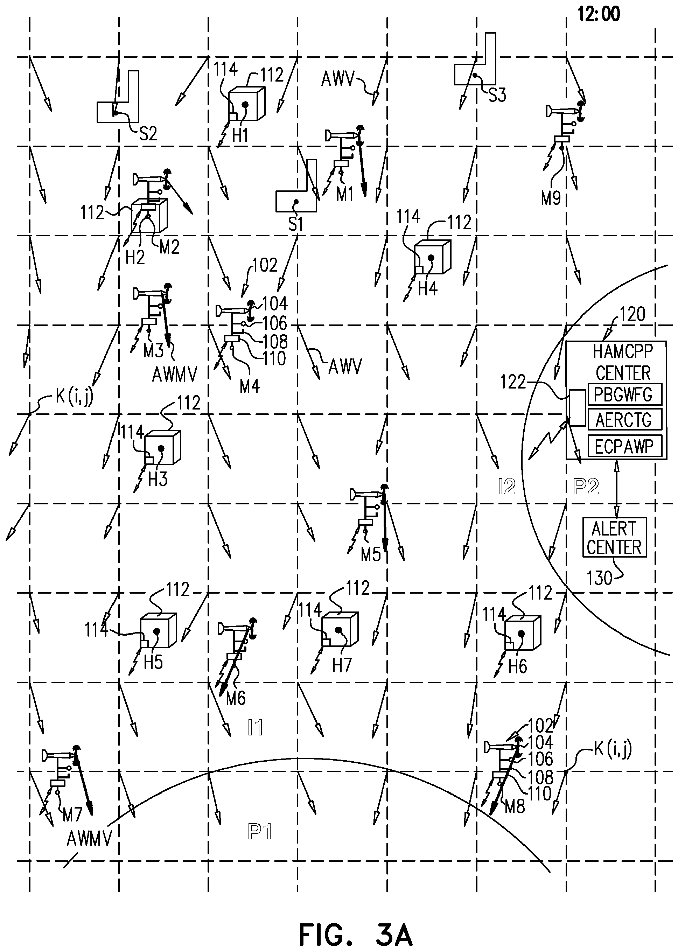

The system described hereinabove preferably operates in an operational mode, here termed OM-A, illustrated in Fig. 2 and in Figs. 3A - 3E. Initially, PBGWFG computes a gridded wind field in the geographical region to be monitored based on average wind velocities received at the HAMCPP center 120 from meteorological stations 102 or calculated by HAMCPP center 120 from inputs received from meteorological stations 102. As will be described hereinbelow in greater detail, the gridded wind field is employed in a calculation of the expected concentrations of hazardous airborne materials in the geographical region to be monitored as a function of an ascertained emission rate of the hazardous airborne materials at a source.

The term "ascertained" as used herein refers to either an emission rate which is assumed to exist, as in the present example of operational mode OM-A, or an emission rate which is calculated, as in operational modes OM-B and OM-C as described hereinbelow.

It is a particular feature of an embodiment of the present invention that for the purposes of each such calculation of expected concentration of a hazardous airborne material, the gridded wind field is assumed to be persistent for an expected time of travel of the hazardous airborne materials to a protected populated area, typically up to 6 hours, and the calculated expected concentrations are employed in alerts or other notifications to relevant authorities.

In an initial step, here termed A-I, a square grid, as illustrated in Fig. 1, is overlaid on the geographical region to be monitored and defines a multiplicity of grid vertices, K(ij), preferably each separated from each adjacent grid vertex K by a grid distance of 3 - 6 kilometers. The grid distances between the vertices K are dependent on the topographical complexity and availability of meteorological stations. The locations S of the sources and the locations M of the meteorological stations 102 are indicated on the overlaid square grid, which locations may be expressed as k(x,y). An additional coordinate z, which is perpendicular to the grid, may represent height.

As seen in Fig. 3A, preferably an average wind vector (AWV) is ascertained for each of the multiplicity of grid vertices K, based on the average measured wind vectors (AMWV) at the meteorological stations 102, preferably by employing the Cressman algorithm, as described in "An Operational Objective Analysis System," Mon. Weather Review 87 (1959). A Cressman algorithm is set forth in equations 1 and 2 hereinbelow. Alternatively, other suitable methods of vector interpolation may be used for this purpose. The grid of calculated vectors is stored for later reference and data reduction.

$$V_x(k,t) = [V_{x1}(t)\ dx_1^{-b} + V_{x2}(t)dx_2^{-b} + \ldots + V_{xm}(t)\ dx_m^{-b}]\ /\ (dx_1^{-b} + dx_2^{-b} + \ldots + dx_m^{-b})\ (1)$$

$$V_y(k,t) = [V_{y1}(t)\ dy_1^{-b} + V_{y2}(t)dy_2^{-b} + \ldots + V_{ym}(t)\ dy_m^{-b}]\ /\ (dy_1^{-b} + dy_2^{-b} + \ldots + dy_m^{-b})\ (2)$$

Where:

k is the location of a grid vertex K;

t is the time of the latest wind measurement included in the moving average;

$V_x$ and $V_y$ are the calculated x and y components of the average wind vector, AWV, calculated at grid vertex K at time t;

$V_{x1}$, $V_{x2}$, ..., $V_{xm}$ and $V_{y1}$, $V_{y2}$, ..., $V_{ym}$ are, respectively, the x and y components of the average measured wind vectors (AMWV), based on the actual measured velocities at the locations M of the meteorological stations 102, which x and y components are here designated by indices, 1, 2 ... corresponding to the above designations M1, M2;

$dx_1$, $dx_2$, ..., $dx_m$ and $dy_1$, $dy_2$, ..., $dy_m$ are, respectively, the respective x and y distances between the locations M of each of the meteorological stations 102 and grid vertex K for each grid vertex K; and

b is a distance calibration factor, which is typically set to a value ≥1.

Fig. 3A shows an example of a gridded wind field, based on wind measurements taken at a nominal time of 12:00 noon on January 1, 2016. Locations M1, M2, M3, M4, M5, M6, M7, M8 and M9 of meteorological stations 102, hazardous airborne material source locations, designated by respective designators S1, S2 and S3, and the locations of hazardous airborne material sensor assemblies 112, designated by respective designators H1, H2, H3, H4, H5, H6 and H7, are also indicated in Fig. 3A.

Preferably, each time a new wind velocity is measured, it is assumed that at the time of that measurement a source, located at S, initially emits hazardous airborne material at an initial emission time, Tiet, and at an assumed emission rate Q. This function is preferably carried out by the AERCTG module of HAMCPP center 120.

The gridded wind field is preferably recomputed every one second and is employed in a subsequent calculation of the expected concentrations of hazardous airborne materials in the geographical region to be monitored. The subsequent calculations are similarly employed in updated alerts or other notifications to relevant authorities.

In a step A-II, a computation of an axis of a potential plume of hazardous airborne material is carried out. The axis of the potential plume is computed by considering a theoretical emission of particles of hazardous airborne material from one of the multiple potential sources of hazardous airborne materials, for example, an ammonium plant, an oil refinery, a food additives plant, a pharmaceutical plant, a nuclear power plant, a compost plant and a sewage treatment plant.

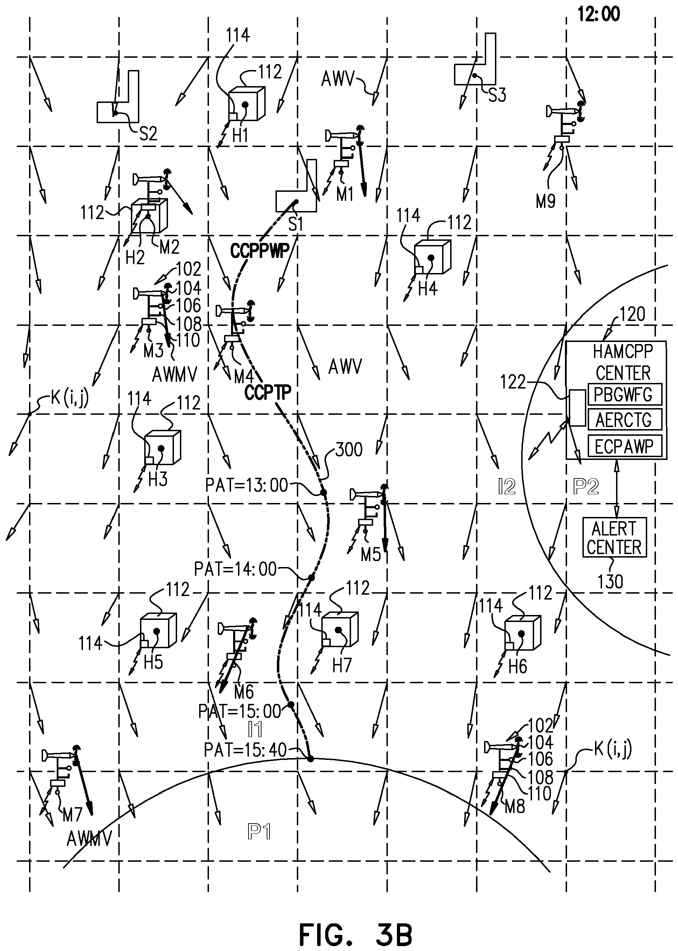

Reference is now made to Fig. 3B, which is identical to Fig. 3A with the addition of a computed axis of a potential plume of hazardous airborne material, which axis is also termed a Computed Central Pathway of Theoretical Particles (CCPTP) and is computed based on the wind velocity measurements taken at 12:00 Noon and assuming wind persistence. The CCPTP is designated by reference numeral 300 and each point $X_a,Y_a$ on the CCPTP 300 is computed preferably in an iterative manner by employing equations (3) and (4)

$$X_a(t+dt) = X_a(t) + V_x(k,t)\ dt \qquad (3)$$

$$Y_a(t+dt) = Y_a(t) + V_y(k,t)\ dt \qquad (4)$$

Where:

dt is a time duration between sequential calculations of the moving average of wind velocity, which is preferably also the time between sequential communications from the meteorological stations 102 to the HAMCPP center 120, typically one second; and

$V_x$ and $V_y$ are the respective x and y components of the average wind vector, AWV, and may be calculated as interpolated wind vectors using the x and y components of the wind velocity at the vertices K of the gridded wind field which vertices K define the grid area in which the calculated point $X_a(t)$, $Y_a(t)$ along the CCPTP 300 was located. Alternatively $V_x$, $V_y$ are calculated at a location k according to equations (1) and (2) hereinabove.

The predetermined time duration over which the moving average is taken is preferably sufficiently long so as to avoid noise resulting from short term phenomena, such as gusts of wind, and is typically at least one minute and less than ten minutes. The moving average is preferably calculated every one second.

The CCPTP 300, which as noted above, is calculated based on an assumption of wind persistence, is also designated as CCPPWP, which indicates that it is based on assumed wind persistence and the current wind data.

It is appreciated that the CCPTP 300 has predicted particle arrival times (PATs) indicated thereon to designate the expected location of particles emitted from source location S at a time Tiet and at various times thereafter, which may be hours after Tiet. In the illustrated example of Fig. 3B, wherein an emission time Tiet at source location S1 at 12:00 noon is assumed, the PATs are typically designated as 13:00, 14:00, 15:00 and 15:40.

An initial segment of the CCPTP 300 is calculated as described above using the x and y coordinates of a given source S1 as $X_a(Tiet)$ and $Y_a(Tiet)$. Each subsequent iteration employs the X(t+dt) and the Y(t+dt) from the previous iteration.

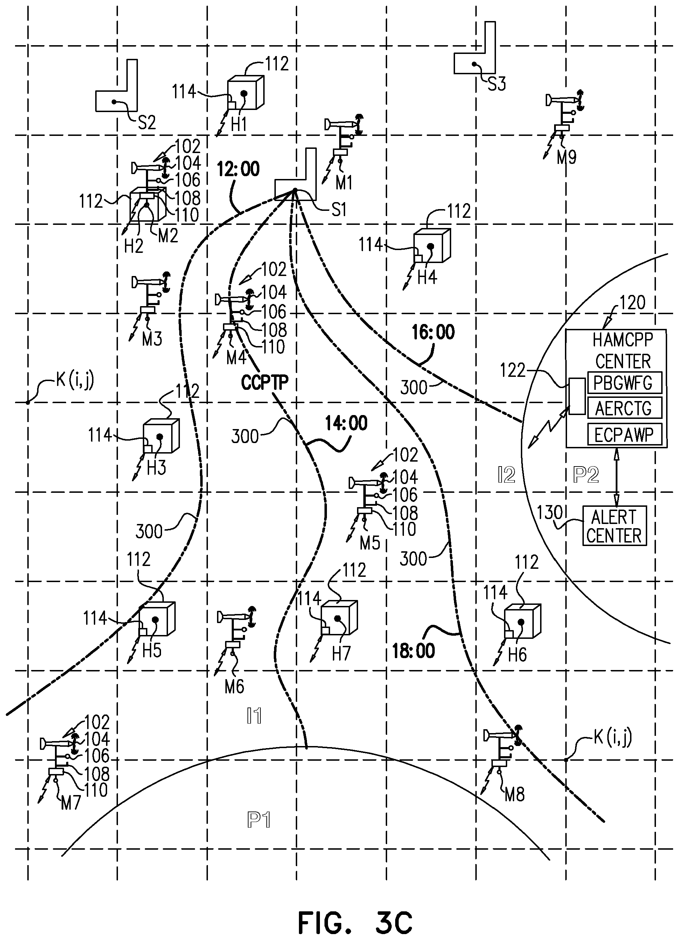

Each of steps A-I & A-II is repeated each time that moving average values based on new wind measurements become available, typically every one second. A collection of CCPTPs 300 generated for the same source S1, for example, based on moving average wind velocities calculated at 12:00 Noon, 14:00, 16:00 and 18:00 on January 1, 2016 appears in Fig. 3C.

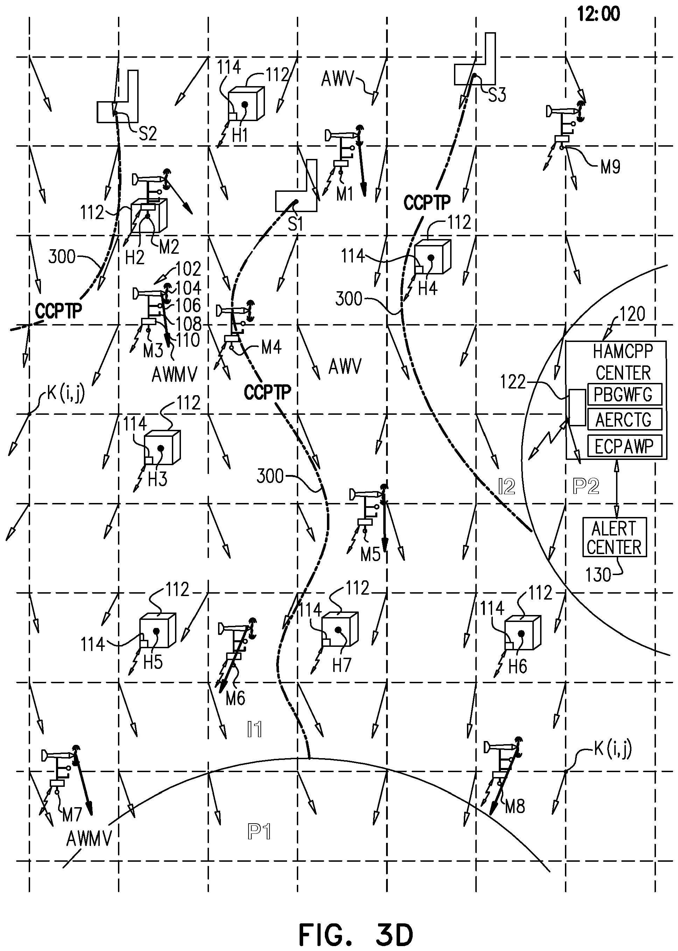

Fig. 3D illustrates CCPTPs 300 for source locations S1, S2 and S3 which are geographically spaced from each other. The CCPTPs 300 are all based on moving averages of wind velocities calculated at 12 Noon on January 1, 2016.

Once a CCPTP 300 has been computed for a given source located at a location S, in step A-II, the concentration of hazardous airborne material at a plurality of grid vertices K, preferably mutually separated by about 100 meters, orthogonally spaced from points along CCPTP 300 is computed, preferably in ECPAWP module of HAMCPP center 120, in a further step A-III, preferably by using the following multi-dimensional equation:

$$C(x,y,z) = (Q / u\pi\sigma_y(t)\ \sigma_z(t)\ )\ exp[-\ (y^2/2\sigma_y(t)^2)\ ]\ exp[-\ (z^2\text{-}G^2)/2\sigma_z(t)^2)]$$

Where:
G is the height of a given source above ground level.

In practice, the concentration is calculated at ground level, i.e. z=0, thus the z direction is ignored and G is assumed to be zero and accordingly the following equation 5 is employed for calculating the concentration of hazardous airborne particles:

$$C(x,y) = (Q / u\pi\sigma_y(t)\ \sigma_z(t))\ exp[-(d_m^2/2\sigma_y(t)^2)] \qquad (5)$$

where:

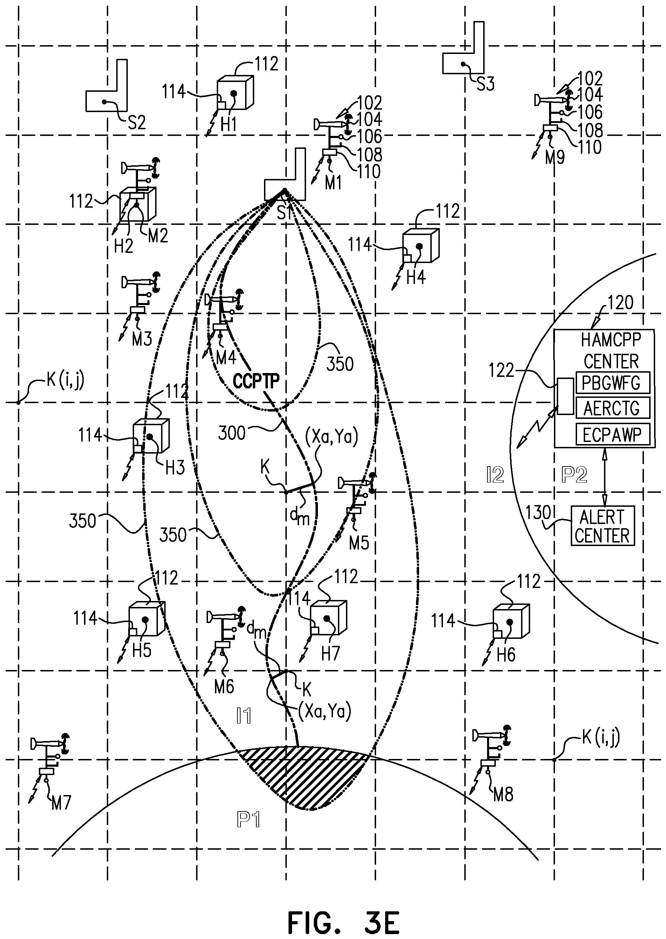

C(x,y) is a calculated concentration at a point x,y, wherein x is downwind with reference to the plume axis CCPTP 300 and $d_m$ is the minimal distance from point x,y to the plume axis CCPTP 300. The point on the plume axis CCPTP 300 which is at the minimal distance $d_m$ from point x,y is designated as point $X_a, Y_a$ in Fig. 3E. The minimum distance $d_m$ from point x,y to the plume axis CCPTP 300 is calculated for each grid vertex K using equation 6 below;
Q is an assumed emission rate at source location;
u is the mean wind speed at point $X_a, Y_a$ along the CCPTP 300 at a given time; and
$\sigma_y(t)$ and $\sigma_z(t)$ are Pasquill diffusion coefficients which depend on the distance along the CCPTP 300 from source location S, for each atmospheric stability level, preferably indicated as a Pasquill category, which may be based on solar radiation levels or on sigma theta and wind speed.

For example, the coordinates $X_a$ and $Y_a$ along the plume axis CCPTP 300 corresponding to a given point K may be determined from equation 6 below which provides the value of $d_m$, the minimum distance of a given point k to the closest point along the CCPTP 300 at a given time:

$$d_m(x,y) = Min\{[(X_a(t) - x_k(t)]^2 + [Y_a(t) - y_k(t)]^2\}^{1/2} \qquad (6)$$

where:

$X_a(t)$ and $Y_a(t)$ are the x,y coordinates in the geographical region to be monitored of a point along the CCPTP 300 at a given time; and
$x_k(t)$ and $y_k(t)$ are the x, y coordinates, respectively, of point k.

The concentrations C(x,y) are preferably calculated for each grid vertex K in the geographical region to be monitored.

It is appreciated that, as noted above, grid vertices K used in step A-III in the calculation of the concentration of hazardous material are preferably mutually separated by about 100 meters, while grid vertices K used in step A-I in the calculation of the gridded wind field, as noted above, are preferably separated by a distance of 3 - 6 kilometers.

In a further step A-IV, iso-concentration lines 350 are computed for a given time, preferably in ECPAWP module of HAMCPP center 120, and displayed as an overlay over a map or other visual representation of the geographical region to be monitored, as shown in Fig. 3E.

Upon receipt of the particle arrival time (PAT) and of the iso-concentration lines 350 displayed as an overlay over a map of the geographical region to be monitored, and receipt of notification of a relevant actual event, the relevant authorities can make a determination as to which populated areas are expected to be exposed to unacceptable levels of hazardous airborne materials and institute suitable warning and other protective measures.

It is appreciated that sensor assemblies 112 may provide an indication to HAMCPP center 120 that a given level of hazardous airborne material has been sensed. This indication typically does not include a quantification of concentration, but may occasion issuance of a predetermined warning to a population. Typically, the quantification of concentration is not communicated to HAMCPP center 120. As a further alternative, sensor assemblies 112 may be obviated in Operation Mode OM-A.

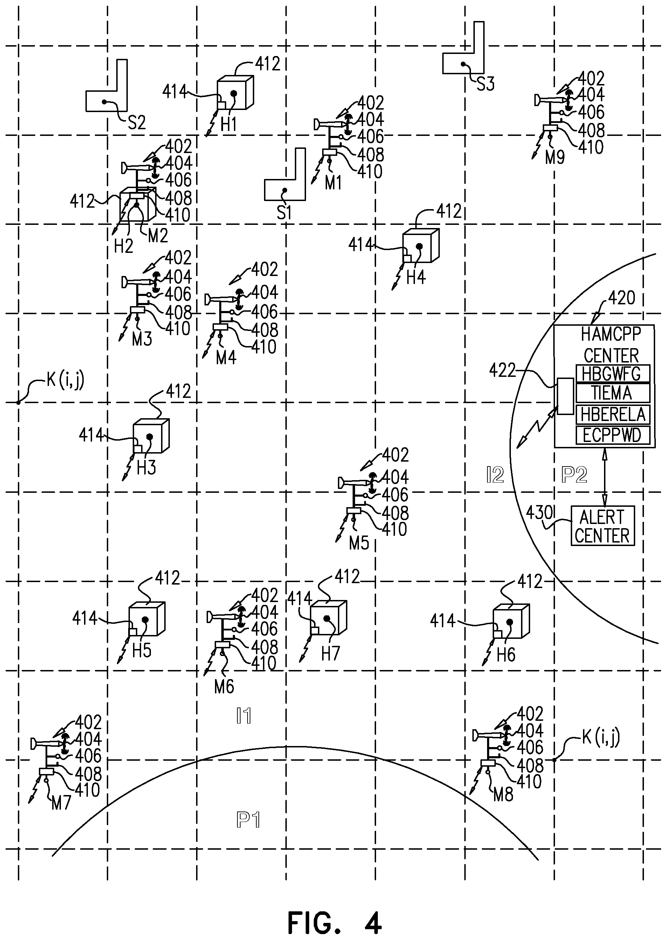

Reference is now made to Fig. 4, which is a simplified illustration of a system for prediction of the presence of hazardous airborne materials, constructed and operative in accordance with yet another preferred embodiment of the present invention.

As seen in Fig. 4, the system preferably operates within a geographical region to be monitored which includes multiple known potential sources of known hazardous airborne materials, for example, an ammonium plant, an oil refinery, a food additives plant, a pharmaceutical plant, a nuclear power plant. a compost plant and a sewage treatment plant, whose locations are known and are designated by respective designators S1, S2, S3 ... Sn, populated areas to be protected, here designated PI, P2 ... Pn and intermediate regions , here designated 11, 12, ... In. It is appreciated that known source locations need not necessarily be fixed locations, for example, a known location of a derailed tanker. For the purposes of the description which follows, it is assumed that the locations S1, S2, S3 are outside of the populated areas to be protected, PI, P2 and separated therefrom by one or more intermediate regions I1, 12.

It is appreciated that not all populated areas are populated areas to be protected. Whether a given populated area is considered to be a populated area to be protected depends, inter alia, on the propinquity of a given source S to the populated area and on the wind conditions at the time of the emission.

The system of Fig. 4 preferably employs a plurality of meteorological stations 402 at known locations in one or more intermediate region, here designated by respective designators M1, M2, M3 ... Mn. Each of the meteorological stations 402 preferably includes a wind velocity and direction measurement device 404 and a pyranometer 406 and may also include temperature, relative humidity and air pressure measurement devices, collectively designated by reference numeral 408. Each of the meteorological stations 402 preferably includes at least one bidirectional communications module 410, which enables communication to and from the meteorological stations 402. Additional meteorological stations 402 may also be located at the source locations S and within the populated areas P to be protected. Alternatively, meteorological data from some of meteorological stations 402 may be obviated by using computer weather forecast models of sufficient resolution and reliability.

The system of Fig. 4 preferably also includes a plurality of geographically dispersed hazardous airborne material sensor assemblies 412, at locations in the intermediate regions, each designated as H1, H2, H3 ... Hn. Hazardous airborne material sensor assemblies 412 may all be identical or, alternatively, different sensors may be specific to different hazardous airborne materials. Hazardous airborne material sensor assemblies 412 preferably each include at least one bidirectional communications module 414, which enables communication of sensor output information from the sensor assembly 412 within the system and also preferably enables remote control of the hazardous airborne material sensor assembly 412.

Sensor assemblies 412 may be co-located with meteorological stations 402 or may be stand-alone. Where a sensor assembly 412 is co-located with a meteorological station 402, communications module 414 may be obviated and the sensor assembly 412 may communicate via communications module 410. Sensor assemblies 412 preferably measure and report the concentrations of hazardous airborne material every one second.

Sensor assemblies 412 each preferably include one or more sensor modules of the types described above with reference to Fig. 1.

In accordance with a preferred embodiment of the present invention, the system includes an Hazardous Airborne Material Concentration Pattern Prediction (HAMCPP) center 420, which may be at any suitable location, fixed or mobile, and may be wholly or partially implemented on the cloud.

HAMCPP center 420 includes a History Based Gridded Wind Field Generator (HBGWFG) module, a Time of Initial Emission of airborne Material Ascertainer (TIEMA) module, an History Based Emission Rate of hazardous airborne material at at least one Emission Location Ascertainer (HBERELA) module, and particle arrival time (PAT) and an Expected Concentration Pattern ascertainer employing Prior measured Wind Data (ECPPWD) module.

HAMCPP center 420 also includes one or more communication modules 422 and preferably communicates with the meteorological stations 402 and with the sensor assemblies 412 using any suitable communication technology and preferably provides an hazardous airborne material concentration pattern prediction (HAMCPP) and particle arrival time (PAT) output. The HAMCPP center 420 may also employ additional wind inputs from available computerized weather forecasting models and may integrate such inputs in its mean calculations which are used for computing the HAMCPP output.

The multiplicity of meteorological stations 402 are preferably distributed over the geographical region to be monitored. Typically, between eight and sixteen meteorological stations 402 are employed to cover an area between 28 X 28 km and 60 x 60km. The optimal density of the meteorological stations 402 depends on local topographical and climatic conditions. Each meteorological station 402 preferably measures and logs the wind velocity, preferably measuring every one second and preferably providing an average wind velocity (AWV) over a predetermined duration, typically ranging from one to ten minutes, and logging moving average values and standard deviations of wind velocity as well as solar radiation intensity. Alternatively, as described below, HAMCPP center 420 calculates the average wind velocity from the meteorological stations 402 inputs.

Preferably, data communication from the sensor assemblies 412 to the HAMCPP center 420 is either initiated by a command from the HAMCPP center 420 or carried out automatically at preset times established by the HAMCPP center 420. Such preset times preferably correspond to times immediately following the logging of average values of wind velocity and their standard deviations as well as the solar radiation intensity.

HAMCPP center 420 typically receives inputs via one or more communication modules 422 from the meteorological stations 402, typically including a wind velocity and direction measurement from wind velocity and direction measurement device 404, and may also receive inputs from hazardous airborne material sensor assemblies 412, typically every one second. In one embodiment of the present invention, HAMCPP center 420 provides a moving average of the wind velocities, AWV, over a predetermined duration, which may range from 1 - 10 minutes, and preferably outputs the moving average of the wind velocities every one second. The output of HAMCPP center 420 preferably indicates the predicted pattern of one or more given concentrations of airborne hazardous materials in the geographical region to be monitored and possibly therebeyond. A typical HAMCPP output is one or more iso-concentration contour lines, overlaid over a map and indicating in which populated areas within the region a dangerous concentration of an airborne hazardous material is currently present or is expected to be present.

Preferably, the HAMCPP center 420 communicates the iso-concentration contours overlaid over a map, via its communication modules 422, to one or more population alert centers 430, each of which is responsible for the safety of the inhabitants of a given area. Each alert center 430 may be operated automatically, semiautomatically or manually to generate an appropriate alert to the relevant population.

Examples of alerts may be sirens, radio, television, sms, email and social network notifications or combinations thereof.

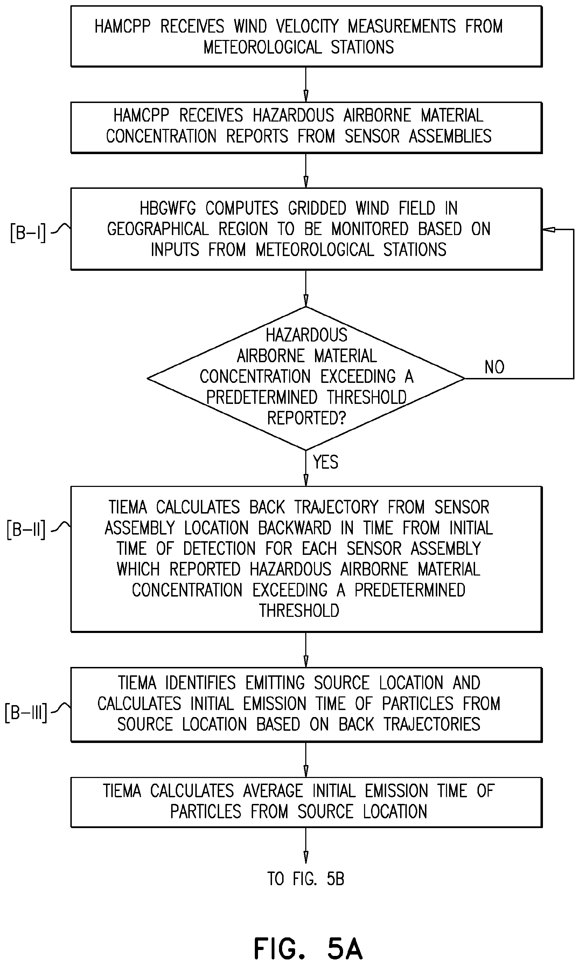

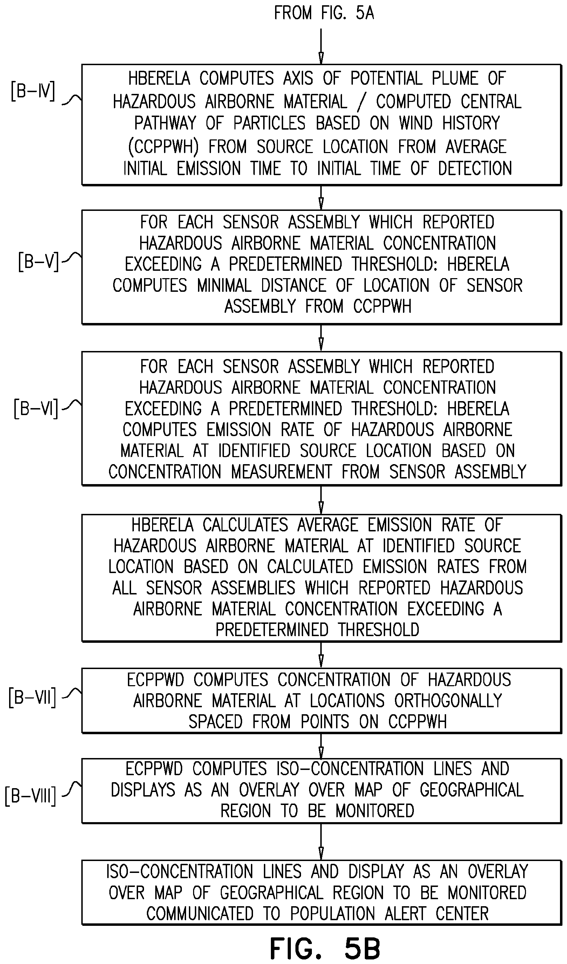

The system described hereinabove preferably operates in an operational mode, here termed OM-B. In this mode of operation, as illustrated in Figs. 4, 5A - 5B and 6A - 6D, initially the HBGWFG computes a gridded wind field in the geographical region to be monitored based on average wind velocities received at the HAMCPP center 420 from meteorological stations 402 at times up to and including the current time or calculated by HAMCPP center 420 from inputs received from meteorological stations 402.

As will be described hereinbelow in greater detail, the gridded wind field is employed in a calculation of the back trajectory of hazardous airborne material, the concentration level of which was found to be above a predetermined threshold by at least one of the hazardous airborne material sensor assemblies 412. The calculation of the back trajectory enables the location of the source emitting the hazardous airborne material to be identified. Additionally, the initial emission time, Tiet, and emission rate of the hazardous airborne material at the source is ascertained. The gridded wind field is also employed in the calculation of the plume axis of the hazardous airborne material emitted from the identified source location.

The plume axis is employed in the calculation of the expected concentrations of hazardous airborne materials in the geographical region to be monitored as a function of an ascertained emission rate of the hazardous airborne materials at a source.

It is a particular feature of an embodiment of the present invention that for the purposes of each such calculation of expected concentration of a hazardous airborne material, the gridded wind field is based on historical data up to the latest measurement and the calculated expected concentrations are employed in alerts or other notifications to relevant authorities.

In an initial step, here termed step B-I, HBGWFG module of HAMCPP center 420 computes a gridded wind field in the geographical region to be monitored based on moving averages, over a predetermined duration, typically ranging from 1 - 10 minutes, of the wind velocities measured by the meteorological stations 402, which are provided, typically every second, to the HAMCPP center 420. The moving averages are taken from the current time, backwards in time.

A square grid is overlaid on the geographical region to be monitored and defines a multiplicity of grid vertices, K(i,j), preferably each separated from each adjacent grid vertex K by a grid distance of 3 - 6 kilometers. The grid distances between the vertices K are dependent on the topographical complexity and availability of meteorological stations. The locations S of the sources, the locations M of the meteorological stations 402 and the locations H of hazardous airborne material sensor assemblies 412 are indicated on the overlaid square grid, which locations may be expressed as k(x,y). An additional coordinate z, which is perpendicular to the grid, may represent height.

Preferably, an average wind vector (AWV) is ascertained for each of the multiplicity of grid vertices K, based on the average measured wind vectors, AMWV, at the meteorological stations 402, preferably by employing the Cressman algorithm, as described in "An Operational Objective Analysis System," Mon. Weather Review 87 (1959), which is incorporated herein by reference. The Cressman algorithm is set forth in equations 1 and 2 hereinabove. Alternatively, other suitable methods of vector interpolation may be used for this purpose. The grid of calculated vectors is stored for later reference and data reduction.

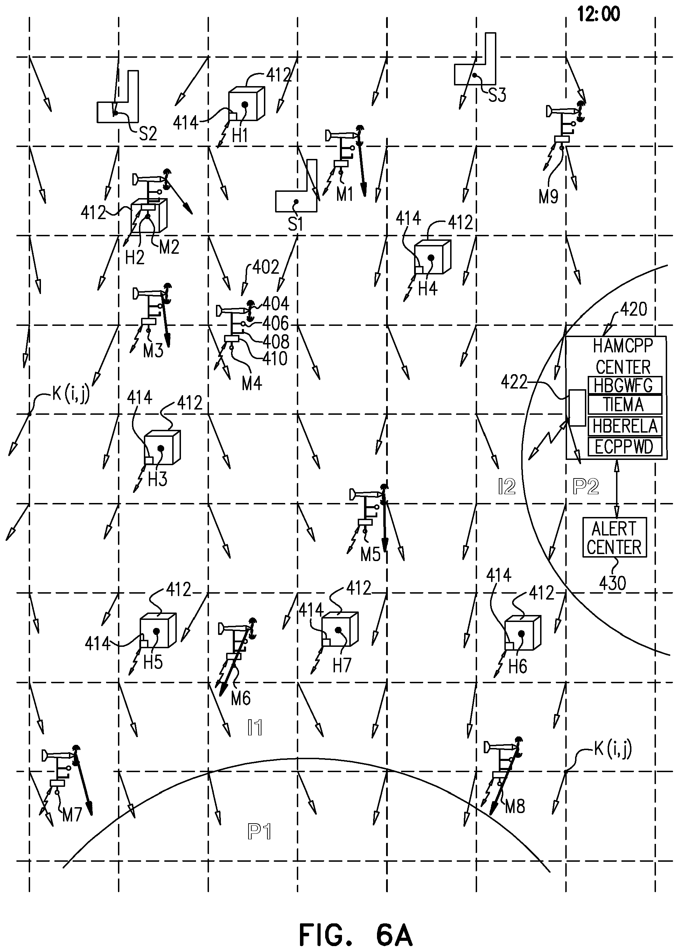

Fig. 6A shows an example of a gridded wind field, based on wind measurements taken at a nominal time of 12:00 noon on January 1, 2016. Locations M1, M2, M3, M4, M5, M6, M7, M8 and M9 of meteorological stations 402, hazardous airborne material source locations, designated by respective designators S1, S2 and S3, and the locations of hazardous airborne material sensor assemblies 412, designated by respective designators H1, H2, H3, H4, H5, H6 and H7, are also indicated in Fig. 6A.

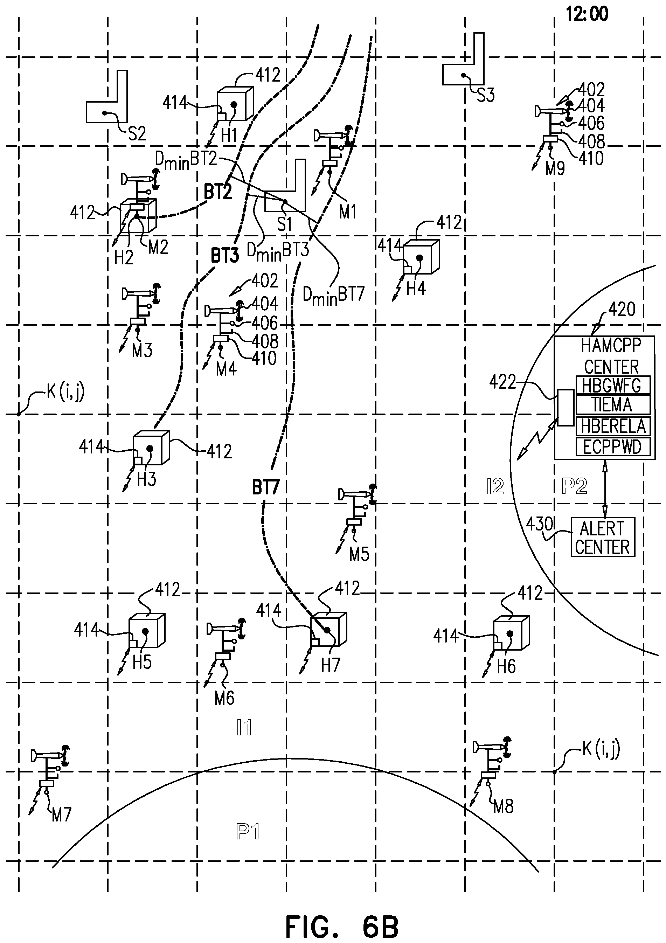

In the event that one or more of hazardous airborne material sensor assemblies 412 detects the existence of a concentration of an hazardous airborne material threat, which concentration exceeds a predetermined threshold, such quantification of hazardous airborne material concentration detection is reported to the HAMCPP center 420. In response to receipt of such a report, the HAMCPP center 420 initiates a further step, designated as step B-II and shown in Fig. 6B, preferably carried out by the TIEMA module of HAMCPP center 420, wherein there is performed a calculation of back trajectories in time of the pathways of particles detected by each of the sensor assemblies 412 which reported hazardous airborne material concentration levels exceeding a predetermined threshold. The back trajectories are preferably calculated back to the locations of possible relevant sources of the hazardous airborne materials. The calculations are based, inter alia, on the gridded wind field as computed based on the latest moving average of the wind velocity.

The back trajectories are each calculated backward from the time that each of the sensor assemblies 412 initially detects the existence of an over-threshold concentration, here termed the "initial detection time" (Tidt). The locations of hazardous airborne material sensor assemblies 412 which reported over-threshold concentrations of hazardous airborne materials are designated in Fig. 6B by designators H2, H3 and H7. The pathways whose back trajectories are calculated are respectively designated in Fig. 6B by designators BT2, BT3 and BT7.

The back trajectory pathway for each of the hazardous airborne material sensor assemblies 412 which reported over-threshold concentrations of hazardous airborne materials is preferably calculated using the following iterative formulas to find $X_{bt}$ and $Y_{bt}$, which are the coordinates (x,y) of each point k on the back trajectory pathway:

$$X_{bt}(t-dt) = X_{bt}(t) - V_x(k,t)\, dt \qquad (7)$$

$$Y_{bt}(t-dt) = Y_{bt}(t) - V_y(k,t)\, dt \qquad (8)$$

where:

dt is a time duration between sequential calculations of the moving average of wind velocity, which is preferably also the time between sequential communications from the meteorological stations 402 to the HAMCPP center 420, typically one second; and

$V_x$ and $V_y$ are interpolated wind vectors using the x and y components of the moving average of wind vectors at the vertices K of the gridded wind field, which vertices K define the grid area in which the previously calculated point $X_{bt}(t)$, $Y_{bt}(t)$ along the back trajectory was located.

The predetermined time duration over which the moving average is taken is preferably sufficiently long so as to avoid noise resulting from short term phenomena, such as gusts of wind, and is typically at least one minute and less than ten minutes. The moving average is preferably calculated every one second.

Equations (7) and (8) are iterated backward in time from the initial detection time, Tidt, in order to find the pathways followed by particles from their source to the sensor assemblies 412 at which they were initially detected. It can be seen in the example shown in Fig. 6B that all pathways are closer to source location S1 than to the other sources located at S2 and S3.

In a further step, here designated step B-III, also carried out by the TIEMA module, the time of initial emission of particles from a source location S, hereafter termed "initial emission time" (Tiet), is ascertained from the closest distance from the source S and the back trajectory pathway of the sensor assembly 412 which initially detected the over-threshold concentration.

In a typical illustrative case, three different sensor assemblies 412, respectively located at locations H2, H3 and H7, each initially detect an over-threshold concentration at a respective given initial detection time (Tidt2, Tidt3, Tidt7) and a respective back trajectory (BT2, BT3, BT7) is calculated by the HAMCPP center 420 for a representative particle detected at each of locations H2, H3 and H7 leading to a source, here designated S1. The initial emission time (TietS1) at source location S1 is calculated for each back trajectory (BT2, BT3, BT7) by sequentially ascertaining the time that a representative particle is at each location along the pathway from a respective sensor assembly 412 to source location S1, determined by each iteration, until the representative particle is at a location closest to source location S1. The time that the representative particle is at a location defining a minimum distance (DminBT2, DminBT3, DminBT7) to source location S1 is deemed to be the initial emission time (TietSIBT2, TietSIBT3, TietS1BT7) for each given back trajectory.

The initial emission times (TietS1BT2, TietS1BT3, TietS1BT7) calculated for each of the back trajectories (BT2, BT3, BT7) are preferably averaged to ascertain an initial emission time (TietS 1) for a given hazardous material at a given source location S1.

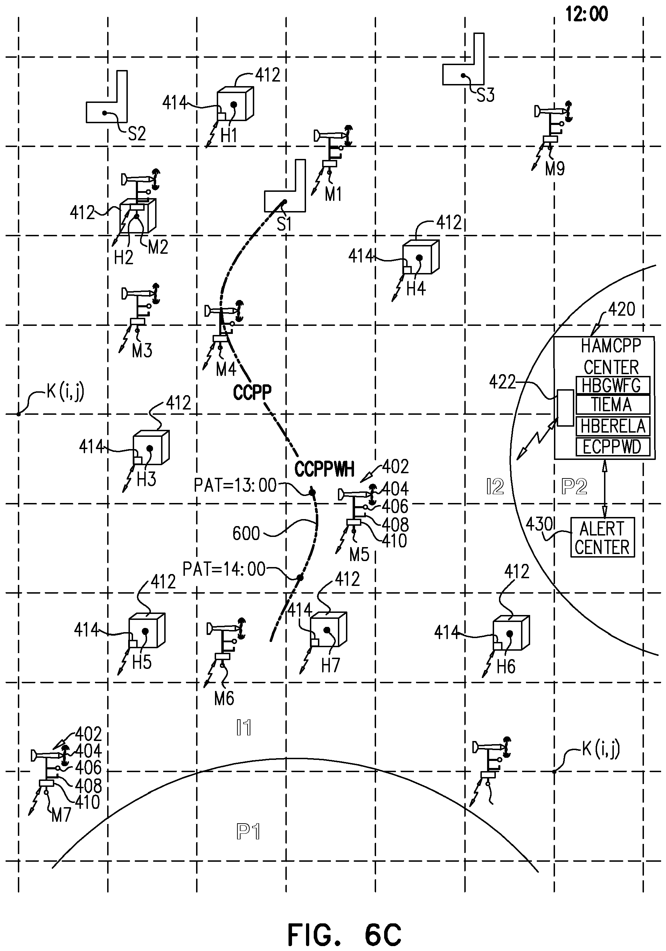

In an additional step, here designated step B-IV and illustrated in Fig. 6C, which is preferably carried out by the HBERELA module of HAMCPP center 420, the axis of a plume of hazardous airborne materials emitted at the initial emission time at a source location S, TietS, is calculated by HAMCPP center 420. The axis of the plume is computed by considering a representative particle in the plume of hazardous airborne materials emitted at a source location S at the initial emission time TietS, based, inter alia, on historical moving average values of the wind velocity measurements taken at time TietS and thereafter. In the present example, the hazardous airborne material is emitted from a source location S1 at time TietS1. The plume axis is computed for times up to Tidt. The calculated plume axis is also referred to as a Computed Central Pathway of Particles based on Wind History (CCPPWH), which indicates that it is based, inter alia, on historical wind data.

The CCPPWH is designated by reference numeral 600 and each point $X_a, Y_a$ on the CCPPWH 600 is computed preferably in an iterative manner by employing equations (9) and (10)

$$X_a(t+dt) = X_a(t) + Vx(k,t) \, dt \qquad (9)$$

$$Y_a(t+dt) = Y_a(t) + Vy(k,t) \, dt \qquad (10)$$

Where:

dt is a time duration between sequential calculations of the moving average of wind velocity, which is preferably also the time between sequential communications from the meteorological stations 402 to the HAMCPP center 420, typically one second; and

$V_x$ and $V_y$ are the respective x and y components of the average wind vector, AWV, and may be calculated as interpolated wind vectors using the x and y components of the wind velocity at the vertices K of the gridded wind field which vertices K define the grid area in which the calculated point $X_a(t)$, $Y_a(t)$ along the CCPPWH 600 was located. Alternatively $V_x$, $V_y$ are calculated at a location k according to equations (1) and (2) hereinabove.

The predetermined time duration over which the moving average is taken is preferably sufficiently long so as to avoid noise resulting from short term phenomena such as gusts of wind, and is typically at least one minute and less than ten minutes. The moving average is preferably calculated every one second.

Equations (9) and (10) are iterated forward in time from the location of the identified source S, starting at the initial emission time, TietS, of the identified source S, up to the initial detection time, Tidt. This iterative process is repeated as new data becomes available in order to find the plume axis CCPPWH 600. As additional sensors 412 detect over-threshold levels of hazardous airborne materials, the accuracy of the calculated initial emission time, TietS, as well as of the CCPPWH 600, is improved.

It is noted that in the present example, as seen in Figs. 6B and 6C, the calculations of the plume axis CCPPWH 600 are based on the coordinates of source S1 and the average time TietS1.

At this stage the emission rate at time TietS1 of the hazardous airborne material at source S1 is ascertained, preferably by the HBERELA module of the HAMCPP center 420.

Preferably, the distance of the sensors assemblies 412, which reported over-threshold emissions of hazardous airborne material originating from source S1, from the central plume axis CCPPWH 600 is calculated.

It is appreciated that the plume axis CCPPWH 600 has computed particle arrival times (PATs) indicated thereon to designate the computed location of particles emitted from source location S at a time Tiet and at various times thereafter, up to and including the initial detection time, Tidt, which may be hours after Tiet. In the illustrated example of Fig. 6C, wherein an emission time Tiet at source location S1 at 12:00 noon is calculated, the PATs are typically designated as 13:00 and 14:00.

In a step B-V, which is preferably carried out by the HBERELA module of HAMCPP center 420, DHPA, the minimum Distance of a given Hazardous airborne material sensor assembly 412, which reported over-threshold emissions of hazardous airborne material originating from the identified source, to Plume Axis CCPPWH 600, is ascertained, preferably, by using equation 11:

$$\mathrm{DHPA}\ (x,y) = \mathrm{Min}\{[(X_a(t) - x_{rs}(t)]^2 + [Y_a(t) - y_{rs}(t)]^2\}^{1/2} \quad (11)$$

where:

$X_a(t)$ and $Y_a(t)$ are the x,y coordinates in the geographical region to be monitored of a point along the CCPPWH 600 at a given time; and
$x_{rs}(t)$, $y_{rs}(t)$ are respectively, the x, y coordinates of a given sensor assembly 412, which reported over-threshold emissions of hazardous airborne material originating from source S.

In the example shown in Fig. 6C, the sensor assemblies 412, which reported over-threshold emissions of hazardous airborne material originating from source S1, are located at locations H2, H3 and H7, and the respective parameters DHPA are designated by $\mathrm{DHPA}_2$, $\mathrm{DHPA}_3$ and $\mathrm{DHPA}_7$.

In a further step B-VI, which is preferably carried out by the HBERELA module of HAMCPP center 420, the emission rate Q of the hazardous airborne material at an identified source, as based on the concentration measurements from a given one of the sensor assemblies 412, which reported over-threshold emissions of hazardous airborne material originating from the identified source, is calculated, using the following equation:

$$Q = (C(x,y,z)\ u\pi\sigma_y(t)\ \sigma_z(t)\ )\ \exp[-\ (\mathrm{DHPA}^2/2\sigma_y(t)^2)]\ \exp[-\ ((z^2-G^2)/2\sigma_y(t)^2)]$$

where:
G is the height of a given source above ground level.

In practice, the concentration is calculated at ground level, i.e. z=0, thus z direction is ignored and G is assumed to be zero, accordingly the following equation 12 is employed for calculating the emission rate of hazardous airborne particles at a given source location S:

$$Q = C(x,y)\ u\pi\sigma_y(t)\ \sigma_z(t)/\ \exp[-\ (\mathrm{DHPA}^2/2\sigma_y(t)^2)] \quad (12)$$

where:

C(x,y) is the concentration measured by a given sensor assembly 412, located at point x,y, which reported over-threshold emissions of hazardous airborne material originating from the identified source;
u is the mean wind speed at a point $X_a$, $Y_a$ along the CCPPWH 600 at a given time;
DHPA is the minimum distance of a given sensor assembly 412 which reported over-threshold emissions of hazardous airborne material originating from the identified source sensor to plume axis CCPPWH 600, as calculated in step B-V above; and
$\sigma_y(t)$ and $\sigma_z(t)$ are Pasquill diffusion coefficients which depend on the distance along the CCPPWH 600 from source location S, for each atmospheric stability level, preferably indicated as a Pasquill category, which may be based on solar radiation levels or on sigma theta and wind speed.

At each iteration an average concentration Qave is calculated and represents the emission rate Q of hazardous airborne material from a given identified source based on the reports of the sensors that reported over-threshold emissions of hazardous airborne material originating from the identified source. Qave may be calculated as a simple average of all the computed values as shown in equation 13, or as a weighted average, for example, inversely proportional to the distance of given sensor assembly 412, which reported over-threshold emissions of hazardous airborne material originating from the identified source, from plume axis CCPPWH 600, as shown in equation 14.

$$\mathrm{Qave} = \Sigma_{i=1}^N\ Q_{rs}/N \quad\quad\quad (13)$$

$$\mathrm{Qave} = \Sigma_{i=1}^N\ \omega_i Q_{rs}/N \quad\quad\quad (14)$$

where:

$Q_{rs}$ is the emission rate of hazardous airborne material emitted from a given identified source, as calculated for each sensor assembly 412, which reported over-threshold concentration of hazardous airborne material originating from the identified source; and

N is the number of sensor assemblies 412, which reported over-threshold concentration of hazardous airborne material originating from the identified source; and

$\omega$ is a weighting factor.

Once the emission rate has been computed for a given identified source at a source location S based on reports of all of the relevant reporting sensors 412, which reported over-threshold emissions of hazardous airborne material originating from the identified source, the concentration of hazardous airborne particles at a plurality of grid vertices K, preferably mutually separated by about 100 meters, is calculated by HAMCPP center 420 in a step B-VII, preferably carried out by the ECPPWD module of HAMCPP center 420 using the following equation (15):

$$C(x,y) = (Qave / u\pi\sigma_y(t)\,\sigma_z(t)\,)\exp[-(d_m{}^2/2\sigma_y(t)^2)] \quad (15)$$

where:

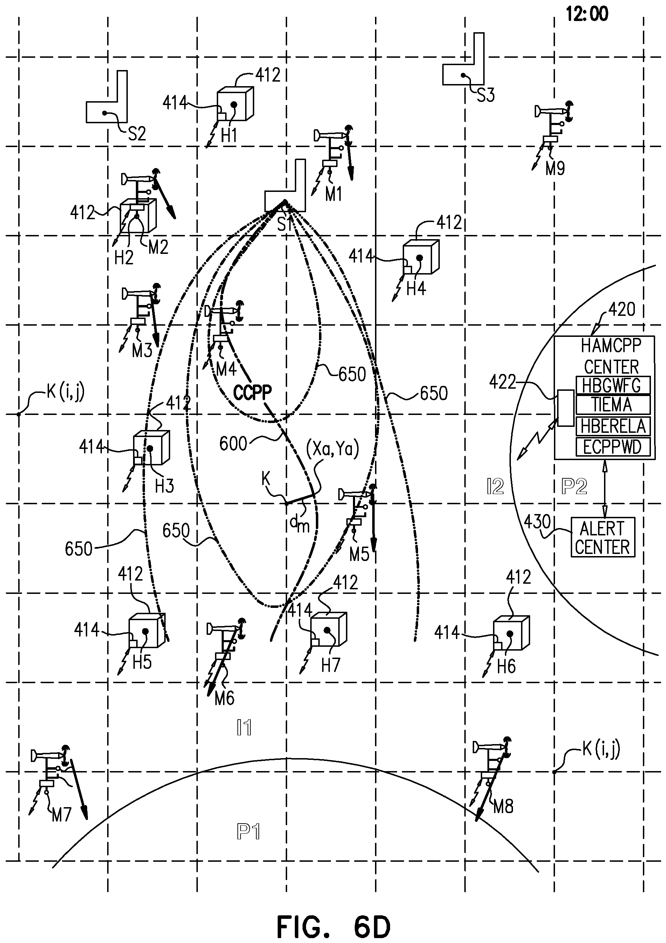

$C(x,y)$ is a calculated concentration at a point x,y, wherein x is downwind with reference to the plume axis CCPPWH;
$d_m$ is the minimal distance from a point x,y, such as a point designated as grid vertex K in Fig. 6D, to the plume axis CCPPWH 600. The point on the plume axis CCPPWH 600 which is at the minimal distance $d_m$ from grid vertex K is designated as $X_a$, $Y_a$ in Fig. 6D. Preferably, the minimum distance $d_m$ from grid vertex K to the plume axis CCPPWH 600 is calculated for each grid vertex K using equation 16 below;
Qave is a calculated average emission rate at source location S, as calculated in equation 13 or equation 14 above;
u is the mean wind speed at a point $X_a$, $Y_a$ along the CCPPWH 600 at a given time; and
$\sigma_y(t)$ and $\sigma_z(t)$ are Pasquill diffusion coefficients which depend on the distance along the CCPPWH 600 from source location S, for each atmospheric stability level, preferably indicated as a Pasquill category, which may be based on solar radiation levels or on sigma theta and wind speed.

For example, the coordinates $X_a$ and $Y_a$ of the closest point on the plume axis CCPPWH 600 to a given grid vertex K may be ascertained using equation 16 below, which provides the value of $d_m$, the minimum distance from a given grid vertex K to the closest point $X_a$, $Y_a$ along the CCPPWH 600 at a given time:

$$d_m(k) = Min\{[(X_a(t) - x_k(t)]^2 + [Y_a(t) - y_k(t)]^2\}^{1/2} \quad (16)$$

where:

$X_a(t)$ and $Y_a(t)$ are the x,y coordinates in the geographical region to be monitored of a point along the CCPPWH 600 at a given time; and
$x_k(t)$, $y_k(t)$ are the x,y coordinates of given grid vertex K.

It is appreciated that, as noted above, grid vertices K used in step B-VII in the calculation of the concentration of hazardous material are preferably mutually separated by about 100 meters, while grid vertices K used in step B-I in the calculation of the gridded wind field, as noted above, are preferably separated by a distance of 3 - 6 kilometers.
In a following step B-VIII, preferably carried out by the ECPPWD module of HAMCPP center 420, iso-concentration lines 650 are calculated and displayed as an overlay over the map or other visual representation of the geographical region to be monitored, as shown in Fig. 6D. The calculation of iso-concentration lines 650 may be entirely conventional and may be carried out using Integrated Data Viewer (IDV) software which can found at http://www.unidata.ucar.edu/software/idv/. It is noted that iso-concentration lines 650 do not necessarily define closed loops.
The iso-concentration lines 650 output by HAMCPP center 420 preferably are updated at predetermined intervals, preferably every one second, and indicate the concentrations of hazardous airborne materials in the geographical region to be monitored and possibly therebeyond. A typical output of HAMCPP center 420 includes one or more iso-concentration contours overlaid over a map and indicating in which populated areas within the region a dangerous concentration of an hazardous airborne material is currently present or is expected to be present. Preferably, the HAMCPP center 420 communicates the iso-concentration contours overlaid over a map to the population alert centers 430.
Upon receipt of the particle arrival time (PAT) and of the iso-concentration lines 650 displayed as an overlay over a map of the geographical region to be monitored, the relevant authorities can make a determination as to which populated areas are in danger in view of unacceptable levels of hazardous airborne materials and institute suitable warning and other protective measures.

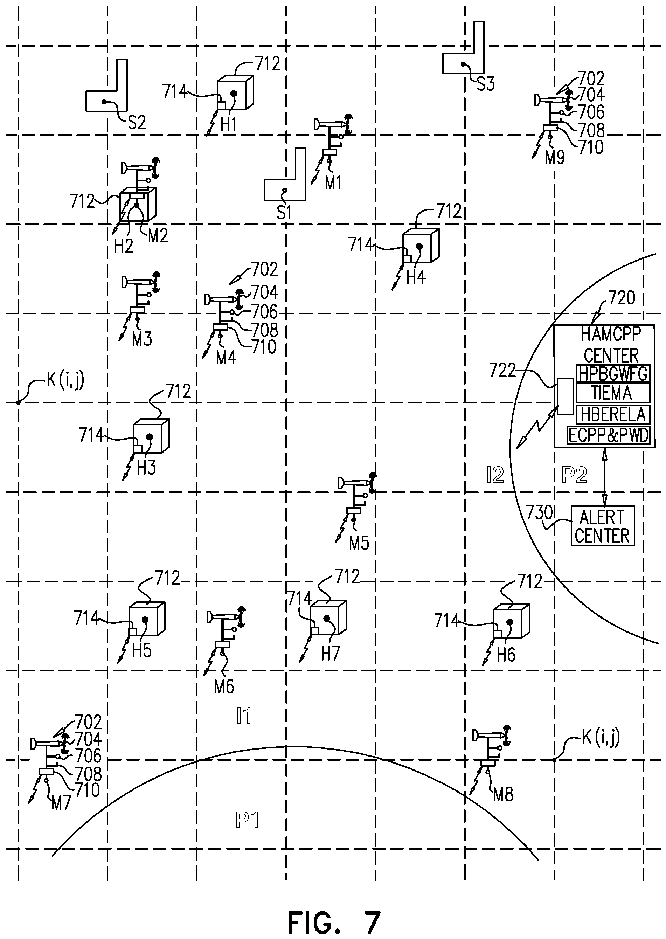

Reference is now made to Fig. 7, which is a simplified illustration of a system for prediction of the presence of hazardous airborne materials, constructed and operative in accordance with yet another preferred embodiment of the present invention.

As seen in Fig. 7, the system preferably operates within a geographical region to be monitored which includes multiple known potential sources of known hazardous airborne materials, for example, an ammonium plant, an oil refinery, a food additives plant, a pharmaceutical plant, a nuclear power plant. a compost plant and a sewage treatment plant, whose locations are known and are designated by respective designators S1, S2, S3 ... Sn, populated areas to be protected, here designated P1, P2 ... Pn, and intermediate regions, here designated I1, 12, ... In. It is appreciated that known source locations need not necessarily be fixed locations, for example a known location of a derailed tanker. For the purposes of the description which follows, it is assumed that the locations S1, S2, S3 are outside of the populated areas to be protected and separated therefrom by one or more intermediate regions.

It is appreciated that not all populated areas are populated areas to be protected. Whether a given populated area is considered to be a populated area to be protected depends, inter alia, on the propinquity of a given source S to the populated area and on the wind conditions at the time of the emission.

It is also appreciated that the system of Fig. 7 may also be employed to protect non-populated areas, such as for protecting agricultural areas from contaminants. It may also be employed for providing accountability for contaminants, such as herbicides, which damage crops and originate from an adjacent or remote location.

The system of Fig. 7 preferably employs a plurality of meteorological stations 702 at known locations in one or more intermediate region, here designated by respective designators M1, M2, M3 ... Mn. Each of the meteorological stations 702 preferably includes a wind velocity and direction measurement device 704 and a pyranometer 706 and may also include temperature, relative humidity and air pressure measurement devices, collectively designated by reference numeral 708. Each of the meteorological stations 702 preferably includes at least one bidirectional communications module 710, which enables communication to and from the meteorological stations 702. Additional meteorological stations 702 may also be located at the source locations S and within the populated areas P to be protected. Alternatively, meteorological data from some of meteorological stations 702 may be obviated by using computer weather forecast models of sufficient resolution and reliability.

The system of Fig. 7 preferably also includes a plurality of geographically dispersed hazardous airborne material sensor assemblies 712, at locations in the intermediate regions, each designated as H1, H2, H3 ... Hn. Hazardous airborne material sensor assemblies 712 may all be identical or, alternatively, different sensors may be specific to different hazardous airborne materials. Hazardous airborne material sensor assemblies 712 preferably each include at least one bidirectional communications module 714, which enables communication of sensor output information from the sensor assembly 712 within the system and also preferably enables remote control of the hazardous airborne material sensor assembly 712.

Sensor assemblies 712 may be co-located with meteorological stations 702 or may be stand-alone. Where a sensor assembly 712 is co-located with a meteorological station 702, communications module 714 may be obviated and the sensor assembly 712 may communicate via communications module 710. Sensor assemblies 712 preferably measure and report the concentrations of hazardous airborne material every one second.

Sensor assemblies 712 each preferably include one or more sensor modules of the types described above with reference to Fig. 1.

In an alternative embodiment, one or more of sensor assemblies 712 may be a portable sensor assembly, such as a sensor located on an emergency vehicle or carried by an emergency worker, such as on a fire truck or by a firefighter. It is appreciated that, in addition to the information communicated by the sensor assemblies described above, the portable sensor assembly also communicates a current location.

In a further alternative embodiment, as noted hereinabove, the system of Fig. 7 may also be employed to protect non-populated areas, such as for protecting agricultural areas from contaminants. In this embodiment, one or more sensor assemblies 712 may be located within the area to be protected, rather than, or in addition to, sensor assemblies 712 located in the intermediate region. It is also appreciated that in this embodiment, upon detection of a concentration of a contaminant above a predetermined threshold, an alert may be provided to an owner of the affected agricultural area, along with the identification of the source of the contamination. The owner may then take appropriate action in near real time or after the fact.

In accordance with a preferred embodiment of the present invention, the system includes an hazardous airborne material concentration pattern prediction (HAMCPP) center 720, which may be at any suitable location, fixed or mobile, and may be wholly or partially implemented on the cloud.

HAMCPP center 720 includes a History and Persistence Based Gridded Wind Field Generator (HPBGWFG) module, a Time of Initial Emission of airborne Material Ascertainer (TIEMA) module, an History Based Emission Rate of hazardous airborne material at at least one Emission Location Ascertainer (HBERELA) module, particle arrival time (PAT) and an Expected Concentration Pattern ascertainer employing Prior measured wind data and Persistence Wind Data based on assumptions of wind persistence (ECPP&PWD) module.

HAMCPP center 720 also includes one or more communication modules 722 and preferably communicates with the meteorological stations 702 and with the sensor assemblies 712 using any suitable communication technology and preferably provides an hazardous airborne material concentration pattern prediction (HAMCPP) and particle arrival time (PAT) output. The HAMCPP center 720 may also employ additional wind inputs from available computerized weather forecasting models and may integrate such inputs in its mean calculations which are used for computing the HAMCPP output.

The multiplicity of meteorological stations 702 are preferably distributed over the geographical region to be monitored. Typically, between eight and sixteen meteorological stations 702 are employed to cover an area between 28 X 28 km and 60 x 60km. The optimal density of the meteorological stations 702 depends on local topographical and climatic conditions. Each meteorological station 702 preferably measures and logs the wind velocity, preferably measuring every one second and preferably providing an average wind velocity (AWV) over a predetermined duration, typically ranging from one to ten minutes, and logging moving average values and standard deviations of wind velocity as well as solar radiation intensity. Alternatively, as described below, HAMCPP center 720 calculates the average wind velocity from the meteorological stations 702 inputs.

Preferably, data communication from the sensor assemblies 712 to the HAMCPP center 720 is either initiated by a command from the HAMCPP center 720 or carried out automatically at preset times established by the HAMCPP center 720. Such preset times preferably correspond to times immediately following the logging of average values of wind velocity and their standard deviations as well as the solar radiation intensity.

The HAMCPP center 720 typically receives inputs via one or more communication modules 722 from the meteorological stations 702, typically including a wind velocity and direction measurement from wind velocity and direction measurement device 704, and may also receive inputs from hazardous airborne material sensor assemblies 712, typically every one second. In one embodiment of the present invention, HAMCPP center 720 preferably provides a moving average of wind velocities, AWV, over a predetermined duration, which may range from 1 - 10 minutes, and preferably outputs the moving average of the wind velocities AWV every one second. The HAMCPP center 720 preferably also employs assumptions regarding wind persistence going forward in time.

A moving average and wind persistence based output of HAMCPP center 720 preferably indicates the predicted pattern of one or more given concentrations of airborne hazardous materials in the geographical region to be monitored and possibly therebeyond. A typical HAMCPP output is one or more iso-concentration contour lines overlaid over a map and indicating in which populated areas within the region a dangerous concentration of an airborne hazardous material is currently present or is expected to be present.

Preferably, the HAMCPP center 720 communicates the iso-concentration contours overlaid over a map, via its communication modules 722, to one or more population alert centers 730, each of which is responsible for the safety of the inhabitants of a given area. Each alert center 730 may be operated automatically, semiautomatically or manually to generate an appropriate alert to the relevant population.

Examples of alerts may be sirens, radio, television, sms, email and social network notifications or combinations thereof.

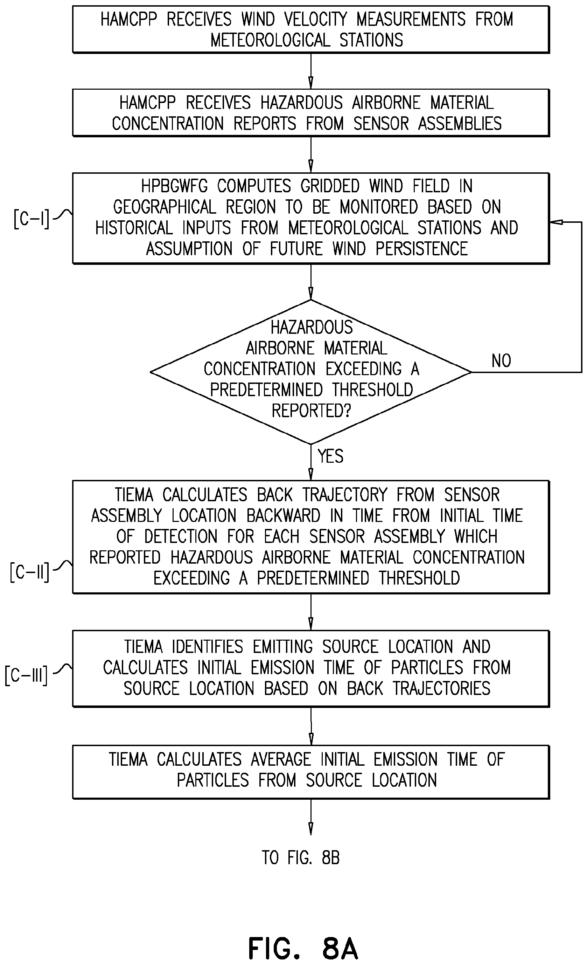

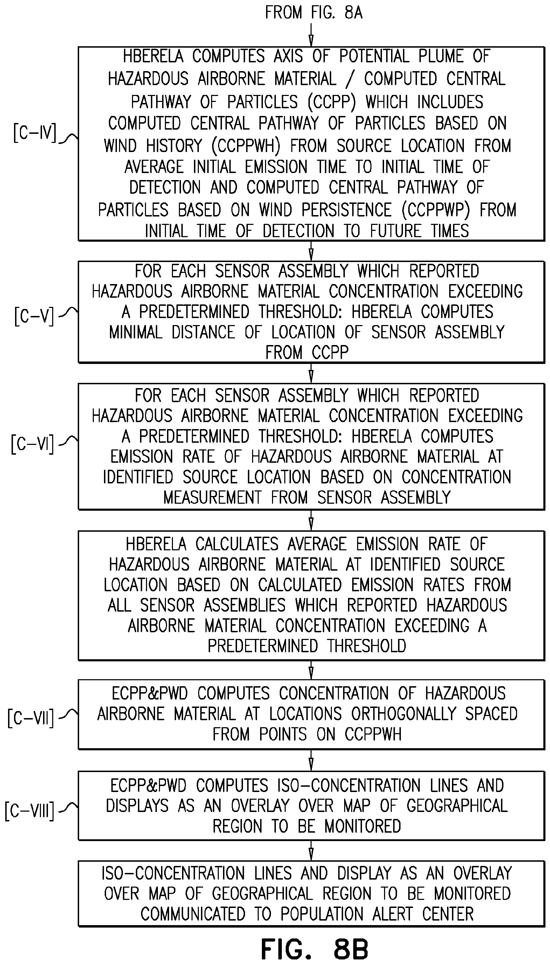

In this mode of operation, here termed OM-C, as illustrated in Figs. 7, 8A - 8B and 9A - 9D, initially the HPBGWFG computes a gridded wind field based on average wind velocities received at the HAMCPP center 720 from meteorological stations 702 at times up to and including the current time or calculated by HAMCPP center 720 from inputs received from meteorological stations 702. All future gridded wind fields are assumed to be identical to the latest stored wind field, based on an assumption of wind persistence.

As will be described hereinbelow in greater detail, the gridded wind field, based on average wind velocities received at the HAMCPP center 720 at times up to and including the current time, is employed in a calculation of the back trajectory of hazardous airborne material, the concentration level of which was found to be above a predetermined threshold by at least one of the hazardous airborne material sensor assemblies 712. The calculation of the back trajectory enables the location of the source emitting the hazardous airborne material to be identified. Additionally, the initial emission time, Tiet, and emission rate of the hazardous airborne material at the source are ascertained. The gridded wind field based on historical wind velocity measurements and on an assumption of wind persistence going forward is also employed in the calculation of the plume axis of the hazardous airborne material emitted from the identified source location. The plume axis is employed in the calculation of the expected concentrations of hazardous airborne materials in the geographical region to be monitored as a function of an ascertained emission rate of the hazardous airborne materials at a source.

It is a particular feature of an embodiment of the present invention that for the purposes of each such calculation of expected concentration of a hazardous airborne material, the gridded wind field is partially based on historical data up to the latest measurement and thereafter to the future is assumed to be persistent for an expected time of travel of the hazardous airborne materials to a protected populated area, typically up to 6 hours, and the calculated expected concentrations are employed in alerts or other notifications to relevant authorities.

In an initial step, here termed step C-I, the HPBGWFG module of HAMCPP center 720 computes a gridded wind field based on average wind velocities received at the HAMCPP center 720 from meteorological stations 702. A square grid is overlaid on the geographical region to be monitored and defines a multiplicity of grid vertices, K(i,j), preferably each separated from each adjacent grid vertex K by a grid distance of 3 - 6 kilometers. The grid distances between the vertices K are dependent on the topographical complexity and availability of meteorological stations. The locations S of the sources and the locations M of the meteorological stations 702 and the locations H of hazardous airborne material sensor assemblies 712 are indicated on the overlaid square grid, which locations, may be expressed as k(x,y). An additional coordinate z, which is perpendicular to the grid, may represent height.

An average wind vector is ascertained for each of the multiplicity of grid vertices K, based on the measured average wind vectors at the meteorological stations 702, preferably by employing the Cressman algorithm, as described in "An Operational Objective Analysis System," Mon. Weather Review 87 (1959), which is incorporated herein by reference. The Cressman algorithm is set forth in equations 1 and 2 hereinabove. Alternatively, other suitable methods of vector interpolation may be used for this purpose. The grid of calculated vectors is stored for later reference and data reduction.

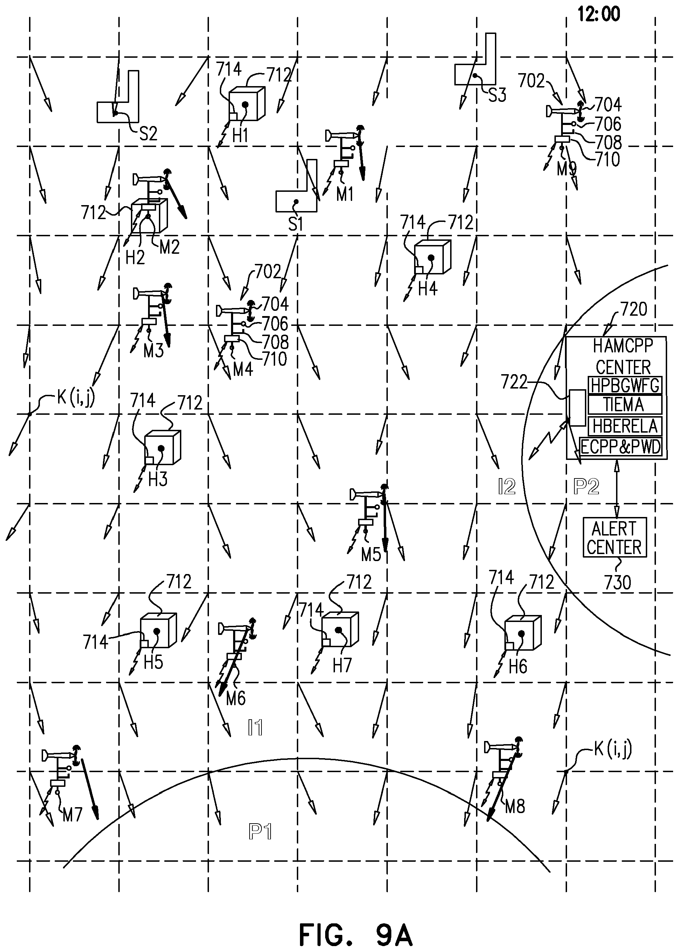

An example of a gridded wind field, based on wind measurements taken at a nominal time of 12 Noon on January 1, 2016, appears in Fig. 9A. Locations M1, M2, M3, M4, M5 and M6 of meteorological stations 702, hazardous airborne material source locations, designated by respective designators S1, S2 and S3, and the locations, designated by respective designators H1, H2, H3, H4, H5, H6 and H7, of hazardous airborne material sensor assemblies 712 are also indicated in Fig. 9A.

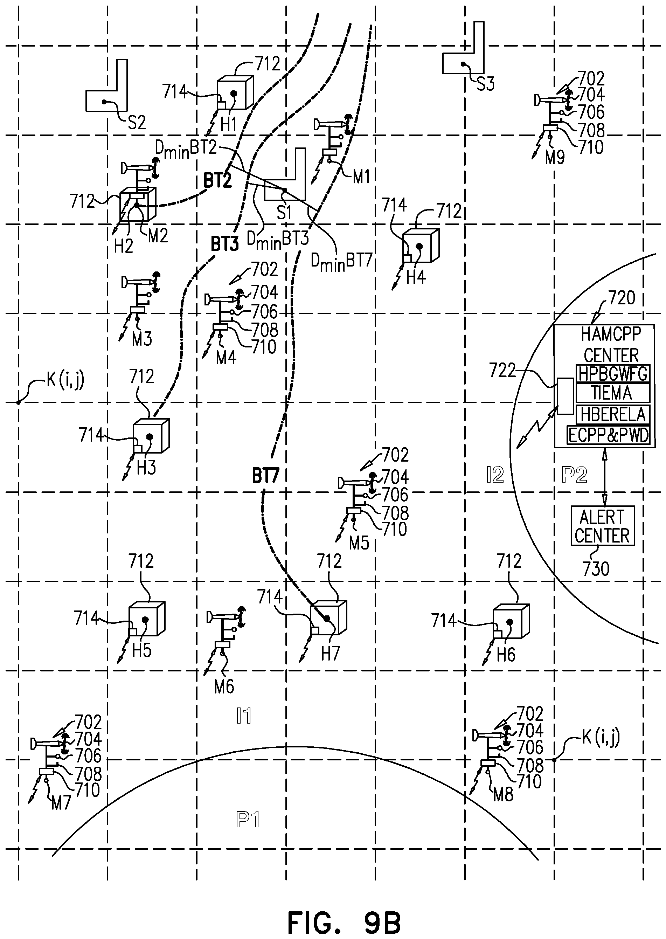

In the event that one or more of hazardous airborne material sensor assemblies 712 detects the existence of a concentration of an hazardous airborne material threat, which concentration exceeds a predetermined threshold, such quantification of hazardous airborne material concentration detection is reported to the HAMCPP center 720. In response to receipt of such a report, the HAMCPP center 720 initiates a further step, designated as step C-II and shown in Fig. 9B, preferably carried out by the TIEMA module of HAMCPP center 720, wherein there is performed a calculation of back trajectories in time of the pathways of particles detected by each of the sensor assemblies 712 which reported hazardous airborne material concentration levels exceeding a predetermined threshold. The back trajectories are preferably calculated back to the locations of possible relevant sources of the hazardous airborne materials. The calculations are based, inter alia, on the gridded wind field as computed based on the latest moving average of the wind velocity

The back trajectories are each calculated backward from the time that each of the sensor assemblies 712 initially detects the existence of an over-threshold concentration, here termed the "initial detection time" (Tidt). The locations of hazardous airborne material sensor assemblies 712 which reported over-threshold concentrations of hazardous airborne materials are designated in Fig. 9B by designators H2, H3 and H7. The pathways whose back trajectories are calculated are respectively designated in Fig. 9B by designators BT2, BT3 and BT7.

The back trajectory pathway for each hazardous airborne material sensor assemblies 712 which reported over-threshold concentrations of hazardous airborne materials is preferably calculated using the following iterative formulas to find $X_{bt}$ and $Y_{bt}$, which are the coordinates (x,y) of each point k on the back trajectory pathway:

$$X_{bt}(t\text{-}dt) = X_{bt}(t) - V_x(k,t)\, dt \qquad (17)$$

$$Y_{bt}(t\text{-}dt) = Y_{bt}(t) - V_y(k,t)\, dt \qquad (18)$$

where:

dt is a time duration between sequential calculations of the moving average of wind velocity, which is preferably also the time between sequential communications from the meteorological stations 702 to the HAMCPP center 720, typically one second; and
$V_x$ and $V_y$ are interpolated wind vectors using the x and y components of the moving average of wind vectors AWV at the vertices K of the gridded wind field, which vertices K define the grid area in which the previously calculated point $X_{bt}(t)$, $Y_{bt}(t)$ along the back trajectory was located.

The predetermined time duration over which the moving average is taken is preferably sufficiently long so as to avoid noise resulting from short term phenomena, such as gusts of wind, and is typically at least one minute and less than ten minutes. The moving average is preferably calculated every one second.

Equations (17) and (18) are iterated backward in time from the initial detection, time, Tidt, in order to find the pathways followed by particles from their source to the sensor assemblies 712 at which they were initially detected. It can be seen in the example shown in Fig. 9B that all pathways are closer to source location S1 than to the other sources located at S2 and S3.

In a further step, here designated step C-III, also carried out by the TIEMA module, the initial emission time of particles from a source location S, hereafter termed "initial emission time" (Tiet), is ascertained from the closest distance from the source S and the back trajectory pathway of the sensor assembly 712 which initially detected the over-threshold concentration.

In a typical illustrative case, three different sensor assemblies 712, respectively located at locations H2, H3 and H7, each initially detect an over-threshold concentration at a respective given initial detection time (Tidt2, Tidt3, Tidt7) and a respective back trajectory (BT2, BT3, BT7) is calculated by the HAMCPP center 720 for a representative particle detected at each of locations H2, H3 and H7 leading to a source, here designated S1. The initial emission time (TietSl) at source location S1 is calculated for each back trajectory (BT2, BT3, BT7) by sequentially ascertaining the time that a representative particle is at each location along the pathway from a respective sensor assembly 712 to source location S1, determined by each iteration, until the representative particle is at a location closest to source location S1. The time that the representative particle is at a location defining a minimum distance (DminBT2, DminBT3, DminBT7) to source location S1 is deemed to be the initial emission time (TietS1BT2, TietS1BT3, TietS1BT7) for each given back trajectory.

The initial emission times (TietS1BT2, TietS1BT3, TietS1BT7) calculated for each of the back trajectories (BT2, BT3, BT7) are preferably averaged to ascertain an initial emission time (TietS1) for a given hazardous material at a given source location S1.

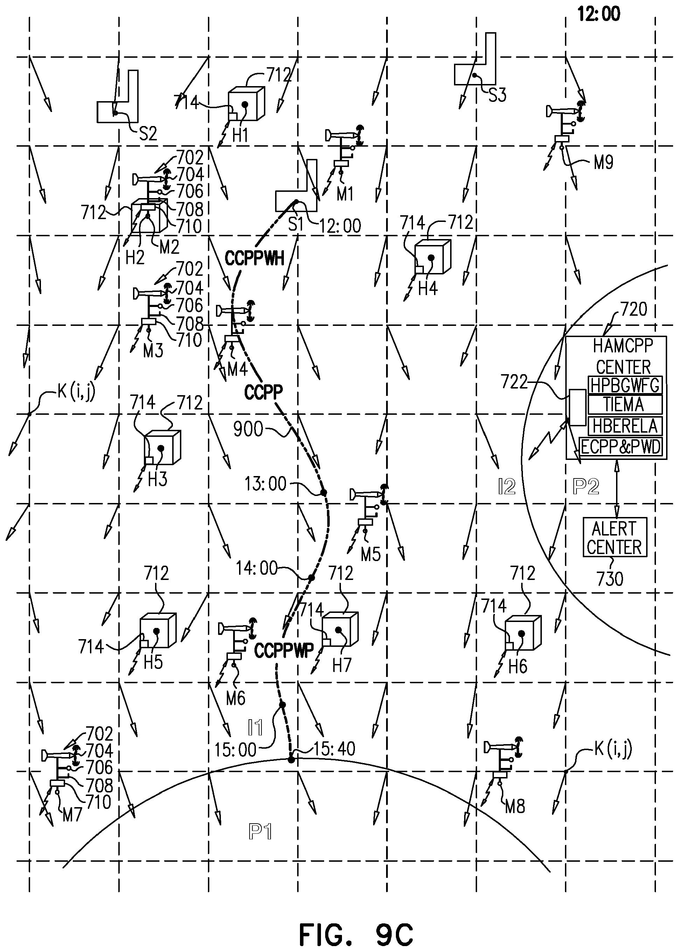

In an additional step, here designated step C-IV and illustrated in Fig. 9C, which is preferably carried out by the HBERELA module of HAMCPP center 720, the axis of a plume of hazardous airborne materials emitted at the initial emission time (TietS) is calculated by HAMCPP center 720. The axis of the plume is computed by considering a representative particle in the plume of hazardous airborne materials emitted at a source location S at the initial emission time TietS, based, inter alia, on historical moving average values of the wind velocity measurements taken at time TietS and, in distinction to operation in mode OM-B, thereafter forward into the future. In the present example, the hazardous airborne material is emitted from a source location S1 at time TietS1. The computation of the plume axis of hazardous airborne material, which is central pathway of the particles (CCPP) emitted at source location S1 from time Tidt forward into the future is based on an assumption of wind persistence.

The portion of the plume axis CCPP which is calculated based on stored historical wind data is designated as Computed Central Pathway of Particles based on Wind History (CCPPWH), which indicates that it is based on historical wind data.

The portion of the plume axis CCPP which is calculated based on an assumption of wind persistence is designated as Computed Central Pathway of Particles based on Wind Persistence (CCPPWP), which indicates that it is based on assumed wind persistence and the current wind data at time Tidt.

The combined plume axis, CCPP, is designated by reference numeral 900 and each point $X_a, Y_a$ on the CCPP 900 is computed preferably in an iterative manner by employing equations (19) and (20)

$$X_a(t+dt) = X_a(t) + Vx(k,t) \, dt \qquad (19)$$

$$Y_a(t+dt) = Y_a(t) + Vy(k,t) \, dt \qquad (20)$$

Where:

dt is a time duration between sequential calculations of the moving average of wind velocity, which is preferably also the time between sequential communications from the meteorological stations 702 to the HAMCPP center 720, typically one second; and
$V_x$ and $V_y$ are the respective x and y components of the average wind vector, AWV, and may be calculated as interpolated wind vectors using the x and y components of the wind velocity at the vertices K of the gridded wind field which vertices K define the grid area in which the calculated point $X_a(t)$, $Y_a(t)$ along the CCPP 900 was located. Alternatively $V_x$, $V_y$ are calculated at a location k according to equations (1) and (2) hereinabove.

The predetermined time duration over which the moving average is taken is preferably sufficiently long so as to avoid noise resulting from short term phenomena such as gusts of wind, and is typically at least one minute and less than ten minutes. The moving average is preferably calculated every one second.

Equations (19) and (20) are iterated forward in time from the location of the identified source S, starting from the initial emission time TietS of the identified source S. This iterative process is repeated as new data becomes available and as a new current gridded wind field is calculated. In each repetition, wind persistence is assumed going forward from the latest calculated gridded wind field based on the latest measured wind data, in order to update the axis of the plume of the hazardous airborne material, CCPP 900. As additional sensors 712 detect over-threshold levels of hazardous airborne materials, the accuracy of the calculated initial emission time, TietS, as well as of the CCPP 900, is improved.

It is noted that in the present example, as seen in Figs. 9B and 9C, the calculations of the plume axis CCPP 900 are based on the coordinates of source S1 and the average time TietS 1.

At this stage the emission rate at time TietS 1 of the hazardous airborne material at source S1 is ascertained, preferably by the HBERELA module of the HAMCPP center 720.

Preferably the distance of the sensors assemblies 712, which reported over-threshold emissions of hazardous airborne material originating from source S1, from the central plume axis CCPP 900 is calculated.

It is appreciated that the plume axis CCPP 900 has both computed and predicted particle arrival times (PATs) indicated thereon to designate the expected location of particles emitted from source location S at a time Tiet and at various times thereafter, both before and after the initial detection time, Tidt, which may be hours after Tiet. In the illustrated example of Fig. 9C, wherein an emission time Tiet at source location S1 at 12:00 noon is calculated, the PATs are typically designated as 13:00, 14:00, 15:00 and 15:40.

In a step C-V, which is preferably carried out by the HBERELA module of HAMCPP center 720, DHPA, the minimum distance of a given Hazardous airborne material sensor assembly 712, which reported over-threshold emissions of hazardous airborne material originating from the identified source, to Plume Axis CCPP 900, is ascertained, preferably, by using equation 21:

$$\mathrm{DHPA(x,y)} = \mathrm{Min}\{[(X_a(t) - x_{rs}(t)]^2 + [Y_a(t) - y_{rs}(t)]^2\}^{1/2} \qquad (21)$$

where:

$X_a(t)$ and $Y_a(t)$ are the x,y coordinates in the geographical region to be monitored of a point along the CCPP 900 at a given time; and

$x_{rs}(t)$, $y_{rs}(t)$ are respectively, the x, y coordinates of a given sensor assembly 712, which reported over-threshold emissions of hazardous airborne material originating from source S .

In the example shown in Fig. 9C, the sensor assemblies 712, which reported over-threshold emissions of hazardous airborne material originating from source S1, are located at locations H2, H3 and H7, and the respective parameters DHPA are designated by $\mathrm{DHPA}_2$, $\mathrm{DHPA}_3$ and $\mathrm{DHPA}_7$.

In a further step C-VI, which is preferably carried out by the HBERELA module of HAMCPP center 720, the emission rate Q of the hazardous airborne material at an identified source, as based on the concentration measurements from a given one of the sensor assemblies 712, which reported over-threshold emissions of hazardous airborne material originating from the identified source, is calculated, using the following equation:

$$Q = (\ C(x,y,z)\ u\pi\sigma_y(t)\ \sigma_z(t)\ )\ /\ \exp[-\ (\mathrm{DHPA}^2/2\sigma_y(t)^2)]\ \exp[-\ (z^2 - G^2)/2\sigma_y(t)^2)]$$

where:

G is the height of a given source above ground level.

In practice, the concentration is calculated at ground level, i.e. z=0, thus z direction is ignored and G is assumed to be zero, accordingly the following equation 22 is employed for calculating the emission rate of hazardous airborne particles at a given source location S:

$$Q = C(x,y)\ u\pi\sigma_y(t)\ \sigma_z(t)/\ \exp[-\ (\mathrm{DHPA}^2/2\sigma_y(t)^2)] \qquad (22)$$

where:

$C(x,y)$ is the concentration measured by a given sensor assembly 712, located at point k, which reported over-threshold emissions of hazardous airborne material originating from the identified source;

u is the mean wind speed at a point $X_a$, $Y_a$ along the CCPP 900 at a given time;

DHPA is the minimum distance of a given sensor assembly 712, which reported over-threshold emissions of hazardous airborne material originating from the identified source sensor to plume axis CCPP 900, as calculated in step C-V above;

$\sigma_y(t)$ and $\sigma_z(t)$ are Pasquill diffusion coefficients which depend on the distance along the CCPP 900 from source location S, for each atmospheric stability level, preferably indicated as a Pasquill category, which may be based on solar radiation levels or on sigma theta and wind speed;

At each iteration an average concentration Qave is calculated and represents the emission rate Q of hazardous airborne material from a given identified source based on the reports of the sensors that reported over-threshold emissions of hazardous airborne material originating from the identified source. Qave may be calculated as a simple average of all the computed values as shown in equation 23, or as a weighted average, for example, inversely proportional to the distance of given sensor assembly 712, which reported over-threshold emissions of hazardous airborne material originating from the identified source sensor from plume axis CCPPWH, as shown in equation 24.

$$Qave = \Sigma_{i=1}^{N} Q_{rs}/N \qquad (23)$$

$$Qave = \Sigma_{i=1}^{N} \omega_i Q_{rs}/N \qquad (24)$$

where:

$Q_{rs}$ is the emission rate of hazardous airborne material emitted from a given identified source, as calculated for each sensor assembly 712, which reported over-threshold concentration of hazardous airborne material originating from the identified source;

N is the number of sensor assemblies 712, which reported over-threshold concentration of hazardous airborne material originating from the identified source; and

$\omega$ is a weighting factor.

Once the emission rate has been computed for a given identified source at a source location S based on reports of all of the relevant reporting sensors 712, which reported over-threshold emissions of hazardous airborne material originating from the identified source, the concentration of hazardous airborne particles at a plurality of grid vertices K, preferably mutually separated by about 100 meters, is calculated by HAMCPP center 720 in a step C-VII, preferably carried out by the ECPP&PWD module of HAMCPP center 720 using the following equation (25):

$$C(x,y) = (Qave / u\pi\sigma_y(t) \, \sigma_z(t) \,) \exp[- (d_m^2/2\sigma_y(t)^2)] \quad (25)$$

where:

C(x,y) is a calculated concentration at a point x,y, wherein x is downwind with reference to the plume axis CCPP 900;

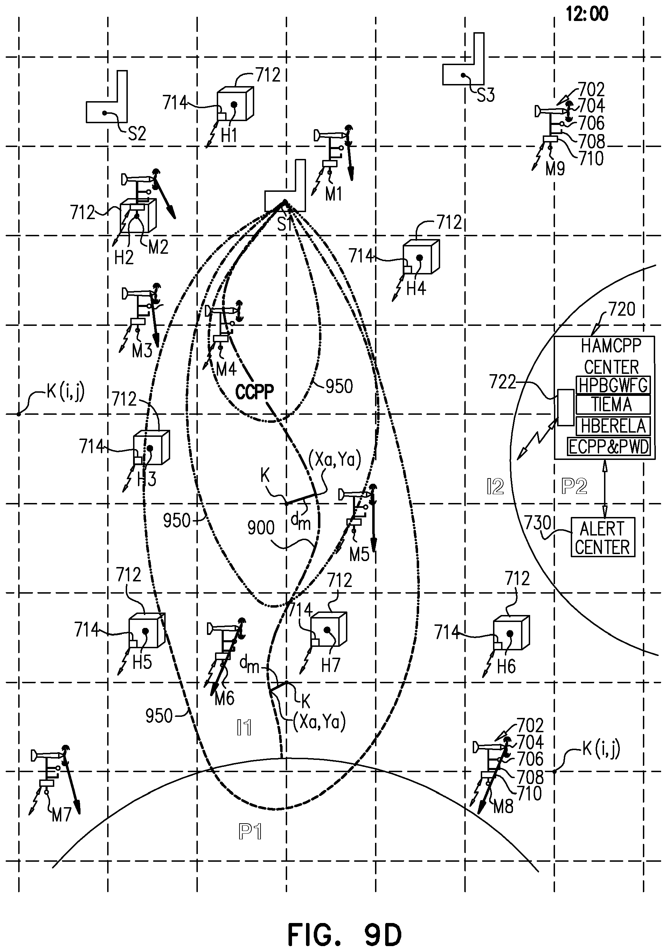

$d_m$ is the minimal distance from point x,y, such as a point designated as grid vertex K in Fig. 9D, to the plume axis CCPP 900. The point on the plume axis CCPP 900 which is at the minimal distance $d_m$ from grid vertex K is designated as $X_a$, $Y_a$ in Fig. 9D. Preferably, the minimum distance $d_m$ from grid vertex K to the plume axis CCPP is calculated for each grid vertex K using equation 26 below;

Qave is a calculated average emission rate at source location S, as calculated in equation 23 or equation 24 above;

u is the mean wind speed at a point $X_a$, $Y_a$ along the CCPP 900 at a given time;

$\sigma_y(t)$ and $\sigma_z(t)$ are Pasquill diffusion coefficients which depend on the distance along the CCPP 900 from source location S, for each atmospheric stability level, preferably indicated as a Pasquill category, which may be based on solar radiation levels or on sigma theta and wind speed.

For example, the coordinates $X_a$ and $Y_a$ of the closest point on the plume axis CCPP 900 to a given grid vertex K may be ascertained using equation 26 below, which provides the value of $d_m$, the minimum distance of a given grid vertex K to the closest point $X_a$, $Y_a$ along the CCPP 900 at a given time:

$$d_m(k) = Min\{[(X_a(t) - x_k(t)]^2 + [Y_a(t) - y_k(t)]^2\}^{1/2} \qquad (26)$$

where:

X_a(t) and Y_a(t) are the x,y coordinates in the geographical region to be monitored of a point along the CCPPWH 900 at a given time; and

x_k(t), y_k(t) are the x,y coordinates of given grid vertex K.

It is appreciated that, as noted above, grid vertices K used in step C-VII in the calculation of the concentration of hazardous material are preferably mutually separated by about 100 meters, while grid vertices K used in step C-I in the calculation of the gridded wind field, as noted above, are preferably separated by a distance of 3 - 6 kilometers.

In a following step C-VIII, preferably carried out by the ECPP&PWD module of HAMCPP center 720, iso-concentration lines 950 are calculated and displayed as an overlay over the map or other visual representation of the geographical region to be monitored, as shown in Fig. 9D. The calculation of iso-concentration lines 950 may be entirely conventional and may be carried out using Integrated Data Viewer (IDV) software which can found at http://www.unidata.ucar.edu/software/idv/. It is noted that iso-concentration lines 950 do not necessarily define closed loops.

The iso-concentration lines 950 and particle arrival time PAT output by HAMCPP center 720 preferably are updated at predetermined intervals, preferably every one second, and indicate the concentrations of hazardous airborne materials in the geographical region to be monitored and possibly therebeyond. A typical output of HAMCPP center 720 includes one or more iso-concentration contours overlaid over a map and indicating in which populated areas within the region a dangerous concentration of an hazardous airborne material is currently present or is expected to be present. Preferably, the HAMCPP center 720 communicates the iso-concentration contours overlaid over a map to the population alert centers 730.

Upon receipt of the particle arrival time (PAT) and of the iso-concentration lines 950 displayed as an overlay over a map of the geographical region to be monitored, the relevant authorities can make a determination as to which populated areas are in danger in view of unacceptable levels of hazardous airborne materials and institute suitable warning and other protective measures.

It will be appreciated by persons skilled in the art that the present invention is not limited to what has been particularly shown and described hereinabove. Rather the scope of the present invention includes both combinations and subcombinations of various features described hereinabove and modifications thereof, which are not in the prior art.

## Claims

1. A method for predicting presence of hazardous airborne materials in a region to be protected, the method comprising:

   receiving an indication of an actual or possible hazardous airborne material emission at at least one emission location outside of said region to be protected and spaced therefrom by an intermediate region;
   ascertaining said at least one emission location;
   ascertaining a time of initial emission of said airborne material;
   ascertaining an emission rate of hazardous airborne material at said at least one emission location; and
   based on measured wind pattern data taken at multiple mutually displaced wind measurement locations in said intermediate region, and at least said time of initial emission and said emission rate, calculating an expected concentration pattern over time of said hazardous airborne materials.

2. A method for predicting presence of hazardous airborne materials according to claim 1 and wherein said receiving an indication comprises measuring an actual concentration of said hazardous airborne material at a concentration measurement location displaced from said at least one emission location.

3. A method for predicting presence of hazardous airborne materials according to claim 1 or claim 2 and wherein said calculating an expected concentration pattern comprises:
   based on measured wind pattern data taken at multiple mutually displaced wind measurement locations, calculating an axis of an emission plume of said hazardous airborne material from said each of said at least one emission location.

4. A method for predicting presence of hazardous airborne materials according to claim 3 and wherein said calculating an expected concentration pattern further comprises:
   calculating at least one minimum distance of at least one concentration measurement location from said axis of said emission plume.

5. A method for predicting presence of hazardous airborne materials according to any of claims 1 - 4 and wherein said calculating an expected concentration pattern additionally comprises generating iso-concentration lines.

6. A method for predicting presence of hazardous airborne materials according to any of the preceding claims and also comprising providing an alert to persons located or expected to be located within in a region to be protected and expected to encounter a concentration of said hazardous airborne material above a predetermined threshold.

7. A method for predicting presence of hazardous airborne materials according to any of the preceding claims and wherein said calculating an expected concentration pattern over time of said hazardous airborne materials comprises:

   calculating a gridded wind field based on current measured wind velocities; and
   assuming the gridded wind field is persistent for an expected time of travel of the hazardous airborne materials to a protected populated area.

8. A method for predicting presence of hazardous airborne materials according to any of claims 1 - 7 and wherein said calculating an expected concentration pattern over time of said hazardous airborne materials comprises:

   measuring an actual concentration of said hazardous airborne material at at least one concentration measurement location displaced from said at least one emission location;
   calculating a back trajectory from each of said at least one concentration measurement location based on historic measured wind velocities;
   calculating a gridded wind field based on current measured wind velocities; and
   assuming the gridded wind field is persistent for an expected time of travel of the hazardous airborne materials to a protected populated area.

9. A method for predicting presence of hazardous airborne materials according to claim 1 and wherein said ascertaining said at least one emission location comprises:

   measuring an actual concentration of said hazardous airborne material at at least one concentration measurement location displaced from said at least one emission location;
   calculating a back trajectory from each of said at least one concentration measurement location;
   calculating a minimum distance from each of said back trajectories to each of said at least one emission location; and
   selecting an emission location corresponding to a lowest of said minimum distances.

10. A method for predicting presence of hazardous airborne materials according to claim 9 and wherein said ascertaining a time of initial emission of said airborne material comprises:
    selecting a time along said back trajectory corresponding to said lowest of said minimum distances.

11. A system for predicting presence of hazardous airborne materials in a region to be protected, the system comprising:

    a communication module receiving an indication of an actual or possible hazardous airborne material emission at at least one emission location outside of said region to be protected and spaced therefrom by an intermediate region;
    an emission location ascertainer, ascertaining said at least one emission location;
    a time of initial emission ascertainer, ascertaining a time of initial emission of said airborne material;
    an emission rate ascertainer, ascertaining an emission rate of hazardous airborne material at said at least one emission location; and
    an expected concentration pattern generator, operative, based on measured wind pattern data taken at multiple mutually displaced wind measurement locations in said intermediate region and at least said time of initial emission and said emission rate, to calculate an expected concentration pattern over time of said hazardous airborne materials.

12. A system for predicting presence of hazardous airborne materials according to claim 11 and wherein said communication module is operative to receive an actual measured concentration of said hazardous airborne material at a concentration measurement location displaced from said at least one emission location.

13. A system for predicting presence of hazardous airborne materials according to claim 11 or claim 12 and wherein said expected concentration pattern generator is operative, based on measured wind pattern data taken at multiple mutually displaced wind measurement locations, to calculate an axis of an emission plume of said hazardous airborne material from said each of said at least one emission location.

14. A system for predicting presence of hazardous airborne materials according to claim 13 and wherein said expected concentration pattern generator is operative to calculate at least one minimum distance of at least one concentration measurement location from said axis of said emission plume.

15. A system for predicting presence of hazardous airborne materials according to any of claims 11 - 14 and wherein said expected concentration pattern generator also comprises an iso-concentration line generator.

16. A system for predicting presence of hazardous airborne materials according to any of the preceding claims 11 - 15 and also comprising an alert generator operative to provide an alert to persons located or expected to be located within in a region to be protected and expected to encounter a concentration of said hazardous airborne material above a predetermined threshold.

**Patentansprüche**

1. Verfahren zur Vorhersage des Vorhandenseins gefährlicher Stoffe in der Luft in einem zu schützenden Bereich, wobei das Verfahren umfasst:

Empfangen einer Anzeige einer tatsächlichen oder möglichen Emission gefährlichen Stoffs in der Luft an mindestens einer Emissionsposition außerhalb des zu schützenden Bereichs und davon durch einen Zwischenbereich beabstandet;
Ermitteln der mindestens einen Emissionsposition; Ermitteln einer Erstemissionszeit des Stoffs in der Luft; Ermitteln einer Emissionsrate gefährlichen Stoffs in der Luft an der mindestens einen Emissionsposition; und basierend auf gemessenen Windmusterdaten, die an mehreren untereinander versetzten Windmesspositionen in dem Zwischenbereich genommen werden, und auf mindestens der Erstemissionszeit und der Emissionsrate Berechnen eines erwarteten Konzentrationsmusters im zeitlichen Verlauf der gefährlichen Stoffe in der Luft.

2. Verfahren zur Vorhersage des Vorhandenseins gefährlicher Stoffe in der Luft nach Anspruch 1, und wobei das Empfangen einer Anzeige das Messen einer tatsächlichen Konzentration des gefährlichen Stoffs in der Luft an einer Konzentrationsmessposition umfasst, die von der mindestens einen Emissionsposition versetzt ist.

3. Verfahren zur Vorhersage des Vorhandenseins gefährlicher Stoffe in der Luft nach Anspruch 1 oder Anspruch 2 und wobei das Berechnen eines erwarteten Konzentrationsmusters umfasst:
basierend auf gemessenen Windmusterdaten, die an mehreren untereinander versetzten Windmesspositionen genommen werden, Berechnen einer Achse einer Emissionswolke des gefährlichen Stoffs in der Luft von jeder der mindestens einen Emissionsposition.

4. Verfahren zur Vorhersage des Vorhandenseins gefährlicher Stoffe in der Luft nach Anspruch 3 und wobei das Berechnen eines erwarteten Konzentrationsmusters ferner umfasst:
Berechnen mindestens eines Mindestabstands der mindestens einen Konzentrationsmessposition von der Achse der Emissionswolke.

5. Verfahren zur Vorhersage des Vorhandenseins gefährlicher Stoffe in der Luft nach einem der Ansprüche 1 bis 4 und wobei das Berechnen eines erwarteten Konzentrationsmusters zusätzlich das Generieren von Isokonzentrationslinien umfasst.

6. Verfahren zur Vorhersage des Vorhandenseins gefährlicher Stoffe in der Luft nach einem der vorstehenden Ansprüche und außerdem umfassend das Bereitstellen einer Warnung an Personen, die sich in einem zu schützenden Bereich aufhalten oder erwartungsgemäß aufhalten werden, von denen erwartet wird, dass sie auf eine Konzentration des gefährlichen Stoffs in der Luft oberhalb eines vorbestimmten Schwellwerts treffen.

7. Verfahren zur Vorhersage des Vorhandenseins gefährlicher Stoffe in der Luft nach einem der vorstehenden Ansprüche und wobei das Berechnen eines erwarteten Konzentrationsmusters im zeitlichen Verlauf des gefährlichen Stoffs in der Luft umfasst:
Berechnen eines gerasterten Windfelds basierend auf aktuellen gemessenen Windgeschwindigkeiten; und

Annehmen, dass das gerasterte Windfeld für eine erwartete Bewegungszeit der gefährlichen Stoffe in der Luft zu einem geschützten bevölkerten Bereich anhält.

8. Verfahren zur Vorhersage des Vorhandenseins gefährlicher Stoffe in der Luft nach einem der Ansprüche 1 bis 7 und wobei das Berechnen eines erwarteten Konzentrationsmusters im zeitlichen Verlauf der gefährlichen Stoffe in der Luft umfasst:

Messen einer tatsächlichen Konzentration des gefährlichen Stoffs in der Luft an mindestens einer Konzentrationsmessposition, die von der mindestens einen Emissionsposition beabstandet ist;
Berechnen eines Rückwegs von jeder der mindestens einen Konzentrationsmessposition basierend auf historischen gemessenen Windgeschwindigkeiten;
Berechnen eines gerasterten Windfelds basierend auf aktuellen gemessenen Windgeschwindigkeiten; und
Annehmen, dass das gerasterte Windfeld für eine erwartete Bewegungszeit der gefährlichen Stoffe in der Luft zu einem geschützten bevölkerten Bereich anhält.

9. Verfahren zur Vorhersage des Vorhandenseins gefährlicher Stoffe in der Luft nach Anspruch 1 und wobei das Ermitteln der mindestens einen Emissionsposition umfasst:

Messen einer tatsächlichen Konzentration des gefährlichen Stoffs in der Luft an mindestens einer Konzentrationsmessposition, die von der mindestens einen Emissionsposition beabstandet ist;
Berechnen eines Rückwegs von jeder der mindestens einen Konzentrationsmessposition;
Berechnen eines Mindestabstands von jedem der Rückwege zu jeder der mindestens einen Emissionsposition; und
Auswählen einer Emissionsposition entsprechend einem niedrigsten der Mindestabstände.

10. Verfahren zur Vorhersage des Vorhandenseins gefährlicher Stoffe in der Luft nach Anspruch 9 und wobei das Ermitteln einer Erstemissionszeit des gefährlichen Stoffs in der Luft umfasst:
Auswählen einer Zeit entlang des Rückwegs, die dem niedrigsten der Mindestabstände entspricht.

11. System zur Vorhersage des Vorhandenseins gefährlicher Stoffe in der Luft in einem zu schützenden Bereich, wobei das System umfasst:

ein Kommunikationsmodul, das eine Anzeige einer tatsächlichen oder möglichen Emission des gefährlichen Stoffs in der Luft an mindestens einer Emissionsposition außerhalb des zu schützenden Bereichs, der durch einen Zwischenbereich davon beabstandet ist, empfängt;
einen Emissionspositionsermittler, der die mindestens eine Emissionsposition ermittelt; einen Erstemissionszeitermittler, der eine Erstemissionszeit des Stoffs in der Luft ermittelt;
einen Emissionsratenermittler, der eine Emissionsrate gefährlichen Stoffs in der Luft an der mindestens einen Emissionsposition ermittelt; und
einen Generator für ein erwartetes Konzentrationsmuster, der betrieben werden kann, um basierend auf gemessenen Windmusterdaten, die an mehreren untereinander versetzten Windmesspositionen in dem Zwischenbereich genommen werden, und auf mindestens der Erstemissionszeit und der Emissionsrate ein erwartetes Konzentrationsmuster im zeitlichen Verlauf der gefährlichen Stoffe in der Luft zu berechnen.

12. System zur Vorhersage des Vorhandenseins gefährlicher Stoffe in der Luft nach Anspruch 11, und wobei das Kommunikationsmodul betrieben werden kann, um eine tatsächliche Konzentration des gefährlichen Stoffs in der Luft an einer Konzentrationsmessposition zu empfangen, die von der mindestens einen Emissionsposition versetzt ist.

13. System zur Vorhersage des Vorhandenseins gefährlicher Stoffe in der Luft nach Anspruch 11 oder Anspruch 12, und wobei der Generator für ein erwartetes Konzentrationsmuster betrieben werden kann, um basierend auf gemessenen Windmusterdaten, die an mehreren untereinander versetzten Windmesspositionen genommen werden, eine Achse einer Emissionswolke des gefährlichen Stoffs in der Luft von jeder der mindestens einen Emissionsposition zu berechnen.

14. System zur Vorhersage des Vorhandenseins gefährlicher Stoffe in der Luft gemäß Anspruch 13 und wobei der Generator für ein erwartetes Konzentrationsmuster betrieben werden kann, um mindestens einen Mindestabstand von mindestens einer Konzentrationsmessposition von der Achse der Emissionswolke zu berechnen.

**15.** System zur Vorhersage des Vorhandenseins gefährlicher Stoffe in der Luft nach einem der Ansprüche 11 bis 14 und wobei der Generator für ein erwartetes Konzentrationsmuster außerdem einen Isokonzentrationsliniengenerator umfasst.

**16.** System zur Vorhersage des Vorhandenseins gefährlicher Stoffe in der Luft nach einem der vorstehenden Ansprüche 11 bis 15 und außerdem umfassend einen Warnungsgenerator, der betrieben werden kann, um Personen, die sich in einem zu schützenden Bereich aufhalten oder erwartungsgemäß aufhalten werden, von denen erwartet wird, dass sie auf eine Konzentration des gefährlichen Stoffs in der Luft oberhalb eines vorbestimmten Schwellwerts treffen, eine Warnung bereitzustellen.

**Revendications**

**1.** Procédé de prédiction de présence de matériaux dangereux en suspension dans l'air dans une zone devant être protégée, le procédé comprenant :

la réception d'une indication d'une émission effective ou possible de matériaux dangereux en suspension dans l'air au niveau d'au moins un lieu d'émission en dehors de ladite zone devant être protégée et espacé de celle-ci par une zone intermédiaire ;
la confirmation dudit au moins un lieu d'émission ;
la confirmation d'un temps d'émission initiale dudit matériau en suspension dans l'air ;
la confirmation d'un taux d'émission de matériau dangereux en suspension dans l'air au niveau dudit au moins un lieu d'émission ; et
sur la base de données mesurées de modèle de vent prises au niveau de multiples lieux de mesure de vent mutuellement déplacés dans ladite zone intermédiaire, et d'au moins ledit temps d'émission initiale et dudit taux d'émission, le calcul d'un modèle de concentration prévu au fil du temps desdits matériaux dangereux en suspension dans l'air.

**2.** Procédé de prédiction de présence de matériaux dangereux en suspension dans l'air selon la revendication 1 et dans lequel ladite réception d'une indication comprend la mesure d'une concentration effective dudit matériau dangereux en suspension dans l'air au niveau d'un lieu de mesure de concentration déplacé dudit au moins un lieu d'émission.

**3.** Procédé de prédiction de présence de matériaux dangereux en suspension dans l'air selon la revendication 1 ou 2 et dans lequel ledit calcul d'un modèle de concentration prévu comprend :
sur la base de données mesurées de modèle de vent prises au niveau de multiples lieux de mesure de vent mutuellement déplacés, le calcul d'un axe de panache d'émission dudit matériau dangereux en suspension dans l'air depuis chacun dudit au moins un lieu d'émission.

**4.** Procédé de prédiction de présence de matériaux dangereux en suspension dans l'air selon la revendication 3 et dans lequel ledit calcul d'un modèle de concentration prévu comprend en outre :
le calcul d'au moins une distance minimum d'au moins un lieu de mesure de concentration depuis ledit axe dudit panache d'émission.

**5.** Procédé de prédiction de présence de matériaux dangereux en suspension dans l'air selon l'une quelconque des revendications 1 à 4 et dans lequel ledit calcul d'un modèle de concentration prévu comprend en outre la génération de lignes d'iso-concentration.

**6.** Procédé de prédiction de présence de matériaux dangereux en suspension dans l'air selon l'une quelconque des revendications précédentes et comprenant également la fourniture d'une alerte à des personnes situées ou prévues de se situer dans une zone devant être protégée et prévue de présenter une concentration dudit matériau dangereux en suspension dans l'air au-dessus d'un seuil prédéterminé.

**7.** Procédé de prédiction de présence de matériaux dangereux en suspension dans l'air selon l'une quelconque des revendications précédentes et dans lequel ledit calcul d'un modèle de concentration prévu au fil du temps de matériaux dangereux en suspension dans l'air comprend :
le calcul d'un champ de vent aux points de grille sur la base de vitesses actuelles mesurées de vent ; et la supposition que le champ de vent aux points de grille est persistant pendant un temps de déplacement prévu des matériaux dangereux en suspension dans l'air jusqu'à une zone peuplée protégée.

8. Procédé de prédiction de présence de matériaux dangereux en suspension dans l'air selon l'une quelconque des revendications 1 à 7 et dans lequel ledit calcul d'un modèle de concentration prévu au fil du temps desdits matériaux dangereux en suspension dans l'air comprend :

la mesure d'une concentration effective dudit matériau dangereux en suspension dans l'air au niveau d'au moins un lieu de mesure de concentration déplacé depuis ledit au moins un lieu d'émission ;
le calcul d'une trajectoire retour depuis chacun dudit au moins un lieu de mesure de concentration sur la base de vitesses historiques mesurées de vent ;
le calcul d'un champ de vent aux points de grille sur la base de vitesses actuelles mesurées de vent ; et la supposition que le champ de vent aux points de grille est persistant pendant un temps de déplacement prévu des matériaux dangereux en suspension dans l'air jusqu'à une zone peuplée protégée.

9. Procédé de prédiction de présence de matériaux dangereux en suspension dans l'air selon la revendication 1 et dans lequel ladite confirmation dudit au moins un lieu d'émission comprend :

la mesure d'une concentration effective dudit matériau dangereux en suspension dans l'air au niveau d'au moins un lieu de mesure de concentration déplacé depuis ledit au moins un lieu d'émission ;
le calcul d'une trajectoire retour depuis chacun dudit au moins au moins un lieu de mesure de concentration ;
le calcul d'une distance minimum depuis chacune desdites trajectoires retour jusqu'à chacun dudit lieu d'émission ; et
la sélection d'un lieu d'émission correspondant à une plus petite desdites distances minimum.

10. Procédé de prédiction de présence de matériaux dangereux en suspension dans l'air selon la revendication 9 et dans lequel ladite confirmation d'un temps d'émission initiale dudit matériau en suspension dans l'air comprend :
la sélection d'un temps sur ladite trajectoire retour correspondant à ladite plus petite desdites distances minimum.

11. Système de prédiction de présence de matériaux dangereux en suspension dans l'air dans une zone devant être protégée, le système comprenant :

un modèle de communication recevant une indication d'une émission effective ou possible de matériaux dangereux en suspension dans l'air au niveau d'au moins un lieu d'émission en dehors de ladite zone devant être protégée et espacé de celle-ci par une zone intermédiaire ;
un confirmateur de lieu d'émission, confirmant ledit au moins un lieu d'émission ;
un temps de confirmateur d'émission initiale, confirmant un temps d'émission initiale dudit matériau en suspension dans l'air ;
un confirmateur de taux d'émission, confirmant un taux d'émission de matériau dangereux en suspension dans l'air au niveau dudit au moins un lieu d'émission ; et
un générateur de modèle de concentration prévue, opérationnel, sur la base de données mesurées de modèle de vent prises au niveau de multiples lieux de mesure de vent mutuellement déplacés dans ladite zone intermédiaire, et d'au moins ledit temps d'émission initiale et dudit taux d'émission, pour calculer un modèle de concentration prévu au fil du temps desdits matériaux dangereux en suspension dans l'air.

12. Système de prédiction de présence de matériaux dangereux en suspension dans l'air selon la revendication 11 et dans lequel ledit module de communication est opérationnel pour recevoir une concentration mesurée effective dudit matériau dangereux en suspension dans l'air au niveau d'un lieu de mesure de concentration déplacé dudit au moins un lieu d'émission.

13. Système de prédiction de présence de matériaux dangereux en suspension dans l'air selon la revendication 11 ou 12 et dans lequel ledit générateur de modèle de concentration prévu est opérationnel, sur la base de données mesurées de modèle de vent prises au niveau de multiples lieux de mesure de vent mutuellement déplacés, pour calculer un axe de panache d'émission dudit matériau dangereux en suspension dans l'air depuis chacun dudit au moins un lieu d'émission.

14. Système de prédiction de présence de matériaux dangereux en suspension dans l'air selon la revendication 13 et dans lequel ledit générateur de modèle de concentration prévu est opérationnel pour calculer au moins une distance minimum d'au moins un lieu de mesure de concentration depuis ledit axe dudit panache d'émission.

15. Système de prédiction de présence de matériaux dangereux en suspension dans l'air selon l'une quelconque des revendications 11 à 14 et dans lequel ledit générateur de modèle de concentration prévu comprend également un générateur de lignes d'isoconcentration.

16. Système de prédiction de présence de matériaux dangereux en suspension dans l'air selon l'une quelconque des revendications 11 à 15 et comprenant également un générateur d'alerte opérationnel pour fournir une alerte à des personnes situées ou prévues de se situer dans une zone devant être protégée et prévue de présenter une concentration dudit matériau dangereux en suspension dans l'air au-dessus d'un seuil prédéterminé.

FIG. 1

```
┌────────────────────────────────────────────────────┐
│   HAMCPP RECEIVES WIND VELOCITY MEASUREMENTS FROM    │
│              METEOROLOGICAL STATIONS                 │
└────────────────────────────────────────────────────┘
                          │
                          ▼
┌────────────────────────────────────────────────────┐
│         PBGWFG COMPUTES GRIDDED WIND FIELD IN        │
[A-I]    │ GEOGRAPHICAL REGION TO BE MONITORED BASED ON        │
│          INPUTS FROM METEOROLOGICAL STATIONS         │
└────────────────────────────────────────────────────┘
                          │
                          ▼
┌────────────────────────────────────────────────────┐
│       AERCTG COMPUTES AXIS OF POTENTIAL PLUME OF     │
[A-II]   │    HAZARDOUS AIRBORNE MATERIAL / COMPUTED CENTRAL    │
│        PATHWAY OF THEORETICAL PARTICLES (CCPTP)      │
└────────────────────────────────────────────────────┘
                          │
                          ▼
┌────────────────────────────────────────────────────┐
│     ECPAWP COMPUTES CONCENTRATION OF HAZARDOUS       │
[A-III]  │    AIRBORNE MATERIAL AT LOCATIONS ORTHOGONALLY      │
│            SPACED FROM POINTS ON CCPTP               │
└────────────────────────────────────────────────────┘
                          │
                          ▼
┌────────────────────────────────────────────────────┐
│     ECPAWP COMPUTES ISO-CONCENTRATION LINES AND      │
[A-IV]   │   DISPLAYS AS AN OVERLAY OVER MAP OF GEOGRAPHICAL    │
│               REGION TO BE MONITORED                 │
└────────────────────────────────────────────────────┘
                          │
                          ▼
┌────────────────────────────────────────────────────┐
│  ISO-CONCENTRATION LINES AND DISPLAY AS AN OVERLAY   │
│  OVER MAP OF GEOGRAPHICAL REGION TO BE MONITORED     │
│   COMMUNICATED TO POPULATION ALERT CENTER            │
└────────────────────────────────────────────────────┘
```

## FIG. 2

FIG. 3A

FIG. 3B

FIG. 3C

FIG. 3D

FIG. 3E

FIG. 4

HAMCPP RECEIVES WIND VELOCITY MEASUREMENTS FROM METEOROLOGICAL STATIONS

HAMCPP RECEIVES HAZARDOUS AIRBORNE MATERIAL CONCENTRATION REPORTS FROM SENSOR ASSEMBLIES

[B-I] HBGWFG COMPUTES GRIDDED WIND FIELD IN GEOGRAPHICAL REGION TO BE MONITORED BASED ON INPUTS FROM METEOROLOGICAL STATIONS

HAZARDOUS AIRBORNE MATERIAL CONCENTRATION EXCEEDING A PREDETERMINED THRESHOLD REPORTED? — NO

YES

[B-II] TIEMA CALCULATES BACK TRAJECTORY FROM SENSOR ASSEMBLY LOCATION BACKWARD IN TIME FROM INITIAL TIME OF DETECTION FOR EACH SENSOR ASSEMBLY WHICH REPORTED HAZARDOUS AIRBORNE MATERIAL CONCENTRATION EXCEEDING A PREDETERMINED THRESHOLD

[B-III] TIEMA IDENTIFIES EMITTING SOURCE LOCATION AND CALCULATES INITIAL EMISSION TIME OF PARTICLES FROM SOURCE LOCATION BASED ON BACK TRAJECTORIES

TIEMA CALCULATES AVERAGE INITIAL EMISSION TIME OF PARTICLES FROM SOURCE LOCATION

TO FIG. 5B

FIG. 5A

FROM FIG. 5A

[B-IV] — HBERELA COMPUTES AXIS OF POTENTIAL PLUME OF HAZARDOUS AIRBORNE MATERIAL / COMPUTED CENTRAL PATHWAY OF PARTICLES BASED ON WIND HISTORY (CCPPWH) FROM SOURCE LOCATION FROM AVERAGE INITIAL EMISSION TIME TO INITIAL TIME OF DETECTION

[B-V] — FOR EACH SENSOR ASSEMBLY WHICH REPORTED HAZARDOUS AIRBORNE MATERIAL CONCENTRATION EXCEEDING A PREDETERMINED THRESHOLD: HBERELA COMPUTES MINIMAL DISTANCE OF LOCATION OF SENSOR ASSEMBLY FROM CCPPWH

[B-VI] — FOR EACH SENSOR ASSEMBLY WHICH REPORTED HAZARDOUS AIRBORNE MATERIAL CONCENTRATION EXCEEDING A PREDETERMINED THRESHOLD: HBERELA COMPUTES EMISSION RATE OF HAZARDOUS AIRBORNE MATERIAL AT IDENTIFIED SOURCE LOCATION BASED ON CONCENTRATION MEASUREMENT FROM SENSOR ASSEMBLY

HBERELA CALCULATES AVERAGE EMISSION RATE OF HAZARDOUS AIRBORNE MATERIAL AT IDENTIFIED SOURCE LOCATION BASED ON CALCULATED EMISSION RATES FROM ALL SENSOR ASSEMBLIES WHICH REPORTED HAZARDOUS AIRBORNE MATERIAL CONCENTRATION EXCEEDING A PREDETERMINED THRESHOLD

[B-VII] — ECPPWD COMPUTES CONCENTRATION OF HAZARDOUS AIRBORNE MATERIAL AT LOCATIONS ORTHOGONALLY SPACED FROM POINTS ON CCPPWH

[B-VIII] — ECPPWD COMPUTES ISO-CONCENTRATION LINES AND DISPLAYS AS AN OVERLAY OVER MAP OF GEOGRAPHICAL REGION TO BE MONITORED

ISO-CONCENTRATION LINES AND DISPLAY AS AN OVERLAY OVER MAP OF GEOGRAPHICAL REGION TO BE MONITORED COMMUNICATED TO POPULATION ALERT CENTER

## FIG. 5B

FIG. 6A

FIG. 6B

FIG. 6C

FIG. 6D

FIG. 7

EP 3 339 855 B1

```
┌─────────────────────────────────────────────┐
│   HAMCPP RECEIVES WIND VELOCITY MEASUREMENTS FROM   │
│             METEOROLOGICAL STATIONS                 │
└─────────────────────────────────────────────┘
                      │
                      ▼
┌─────────────────────────────────────────────┐
│   HAMCPP RECEIVES HAZARDOUS AIRBORNE MATERIAL       │
│   CONCENTRATION REPORTS FROM SENSOR ASSEMBLIES      │
└─────────────────────────────────────────────┘
                      │
                      ▼
┌─────────────────────────────────────────────┐
│   HPBGWFG COMPUTES GRIDDED WIND FIELD IN            │
│   GEOGRAPHICAL REGION TO BE MONITORED BASED ON      │
│   HISTORICAL INPUTS FROM METEOROLOGICAL STATIONS AND│
│   ASSUMPTION OF FUTURE WIND PERSISTENCE             │
└─────────────────────────────────────────────┘
```
[C-I]

```
                HAZARDOUS
            AIRBORNE MATERIAL
         CONCENTRATION EXCEEDING A          NO
         PREDETERMINED THRESHOLD
                REPORTED?
```

YES

```
┌─────────────────────────────────────────────┐
│   TIEMA CALCULATES BACK TRAJECTORY FROM SENSOR      │
│   ASSEMBLY LOCATION BACKWARD IN TIME FROM INITIAL TIME│
│   OF DETECTION FOR EACH SENSOR ASSEMBLY WHICH       │
│   REPORTED HAZARDOUS AIRBORNE MATERIAL CONCENTRATION│
│   EXCEEDING A PREDETERMINED THRESHOLD              │
└─────────────────────────────────────────────┘
```
[C-II]

```
┌─────────────────────────────────────────────┐
│   TIEMA IDENTIFIES EMITTING SOURCE LOCATION AND     │
│   CALCULATES INITIAL EMISSION TIME OF PARTICLES FROM│
│   SOURCE LOCATION BASED ON BACK TRAJECTORIES        │
└─────────────────────────────────────────────┘
```
[C-III]

```
┌─────────────────────────────────────────────┐
│   TIEMA CALCULATES AVERAGE INITIAL EMISSION TIME OF │
│   PARTICLES FROM SOURCE LOCATION                    │
└─────────────────────────────────────────────┘
```

TO FIG. 8B

FIG. 8A

43

FROM FIG. 8A

[C-IV] HBERELA COMPUTES AXIS OF POTENTIAL PLUME OF HAZARDOUS AIRBORNE MATERIAL / COMPUTED CENTRAL PATHWAY OF PARTICLES (CCPP) WHICH INCLUDES COMPUTED CENTRAL PATHWAY OF PARTICLES BASED ON WIND HISTORY (CCPPWH) FROM SOURCE LOCATION FROM AVERAGE INITIAL EMISSION TIME TO INITIAL TIME OF DETECTION AND COMPUTED CENTRAL PATHWAY OF PARTICLES BASED ON WIND PERSISTENCE (CCPPWP) FROM INITIAL TIME OF DETECTION TO FUTURE TIMES

[C-V] FOR EACH SENSOR ASSEMBLY WHICH REPORTED HAZARDOUS AIRBORNE MATERIAL CONCENTRATION EXCEEDING A PREDETERMINED THRESHOLD: HBERELA COMPUTES MINIMAL DISTANCE OF LOCATION OF SENSOR ASSEMBLY FROM CCPP

[C-VI] FOR EACH SENSOR ASSEMBLY WHICH REPORTED HAZARDOUS AIRBORNE MATERIAL CONCENTRATION EXCEEDING A PREDETERMINED THRESHOLD: HBERELA COMPUTES EMISSION RATE OF HAZARDOUS AIRBORNE MATERIAL AT IDENTIFIED SOURCE LOCATION BASED ON CONCENTRATION MEASUREMENT FROM SENSOR ASSEMBLY

HBERELA CALCULATES AVERAGE EMISSION RATE OF HAZARDOUS AIRBORNE MATERIAL AT IDENTIFIED SOURCE LOCATION BASED ON CALCULATED EMISSION RATES FROM ALL SENSOR ASSEMBLIES WHICH REPORTED HAZARDOUS AIRBORNE MATERIAL CONCENTRATION EXCEEDING A PREDETERMINED THRESHOLD

[C-VII] ECPP&PWD COMPUTES CONCENTRATION OF HAZARDOUS AIRBORNE MATERIAL AT LOCATIONS ORTHOGONALLY SPACED FROM POINTS ON CCPPWH

[C-VIII] ECPP&PWD COMPUTES ISO-CONCENTRATION LINES AND DISPLAYS AS AN OVERLAY OVER MAP OF GEOGRAPHICAL REGION TO BE MONITORED

ISO-CONCENTRATION LINES AND DISPLAY AS AN OVERLAY OVER MAP OF GEOGRAPHICAL REGION TO BE MONITORED COMMUNICATED TO POPULATION ALERT CENTER

FIG. 8B

FIG. 9A

FIG. 9B

12:00

S2
712
714
H1
S3
M9
702
704
706
708
710
712
M1
S1 12:00
714 712
H2 M2
CCPPWH
H4
702
704
706
708
710
720
M3
M4
722
HAMCPP
CENTER
CCPP
HPBGWFG
TIEMA
K(i,j)
714 712
900
HBERELA
ECPP&PWD
H3
13:00
I2 P2
M5
712
712
14:00
712
712
H5
714
CCPPWP
714
H7
H6
714
M6
ALERT
CENTER
I1
730
702
704
706
708
710
15:00 15:40
K(i,j)
M7
M8
P1

FIG. 9C

FIG. 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IL 147725

- US 9110748 B

**Non-patent literature cited in the description**

- An Operational Objective Analysis System. *Mon. Weather Review,* 1959, 87